(19) Europäisches Patentamt
European Patent Office
Office européen des brevets

(11) EP 4 250 305 A1

(12) EUROPEAN PATENT APPLICATION
published in accordance with Art. 153(4) EPC

(43) Date of publication:
**27.09.2023 Bulletin 2023/39**

(21) Application number: **21894920.4**

(22) Date of filing: **25.10.2021**

(51) International Patent Classification (IPC):
***G16C 20/50*** *(2019.01)* ***G16C 20/10*** *(2019.01)*
***G16C 20/30*** *(2019.01)* ***G16C 60/00*** *(2019.01)*

(52) Cooperative Patent Classification (CPC):
**G16C 20/10; G16C 20/30; G16C 20/50; G16C 60/00**

(86) International application number:
**PCT/KR2021/014995**

(87) International publication number:
**WO 2022/108149 (27.05.2022 Gazette 2022/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.11.2020 KR 20200157751**

(71) Applicant: **Cha University Industry-Academic Cooperation Foundation**
**Pocheon-si, Gyeonggi-do 11160 (KR)**

(72) Inventors:
- **CHO, Hea-Young**
  **Seongnam-si, Gyeonggi-do 13488 (KR)**
- **CHOI, Go-Wun**
  **Seongnam-si, Gyeonggi-do 13488 (KR)**
- **CHOI, Eun-Jeong**
  **Seongnam-si, Gyeonggi-do 13488 (KR)**
- **KIM, Ju Hee**
  **Seongnam-si, Gyeonggi-do 13488 (KR)**
- **KANG, Dong Wook**
  **Seongnam-si, Gyeonggi-do 13488 (KR)**

(74) Representative: **Weickmann & Weickmann PartmbB**
**Postfach 860 820**
**81635 München (DE)**

# (54) PHYSIOLOGICALLY-BASED PHARMACOKINETIC CORE MODEL

(57) One aspect provides a physiologically based pharmacokinetic core model which may be commonly applied to two or more compounds that share the same core structure, and a method of constructing the physiologically based pharmacokinetic core model. According to the physiologically based pharmacokinetic core model and the method of constructing the physiologically based pharmacokinetic core model, there is the effect of conducting exposure evaluation simply only by deriving a distribution coefficient, without constructing a separate PBPK model for each individual compound.

FIG. 12

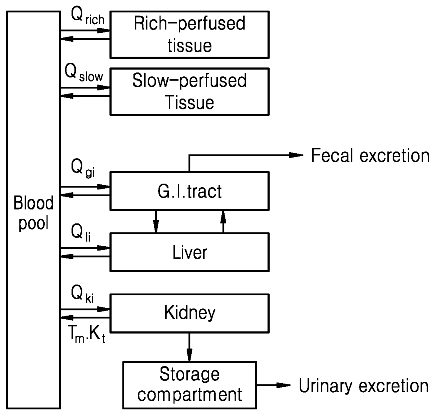




Processed by Luminess, 75001 PARIS (FR)

**Description**

Technical Field

The present disclosure relates to a method of constructing a physiologically based pharmacokinetic (PBPK) core model and a core model of perfluorinated chemicals (PFCs).

Background Art

A compartment model is useful for interpreting blood drug concentration and urine excretion data to explain pharmacokinetics or set a drug administration plan. However, compartments in the model have very limited anatomical representation of real tissues, and connections between the compartments are made by virtual rate constants rather than blood flow, which limits the ability to reflect various variables in real life.

To address these limitations, physiologically-based pharmacokinetic (PBPK) models are being used. A PBPK model is useful for evaluating the risk of hazardous substances from biomonitoring results obtained from the human body by scientifically identifying the correlation between internal and external doses. In exposure evaluation, internal dose or internal equivalent dose refers to the amount of a substance that penetrates the absorption barrier of an organism (for example, skin, lung tissue, gastrointestinal tract) and is absorbed through physical or biological processes, while external dose refers to the amount of a substance exposed to or ingested by a person or animal. Unlike compartment models, PBPK models have the advantage of taking into account the anatomy, physiology, and chemistry of the actual body tissues and organs in order to interpret drug pharmacokinetics. The PBPK model may be used to evaluate exposure levels of hazardous substances, which is difficult to perform directly on humans, or the distribution to target organs through the residual concentration in the human body.

However, existing PBPK models require building a model for each individual compound to be analyzed, and so far, individual PBPK models have been built for each substance, but the development of PBPK models requires a lot of time and various types of experiments, and the development of individual PBPK models for multiple hazardous substances is extremely time-consuming and expensive. Therefore, the development of individual models for all hazardous substances had cumbersome limitations.

In particular, the perfluorinated chemical (PFC) family of hazardous substances has about 20 types of known hazardous substances and is used in the manufacture of fabrics, carpets, paper coatings, cosmetics, various other products, etc., which has increased the risk of widespread exposure to the human body in modern society.

Therefore, the inventors of the present application have solved the above-described problems by developing a method of classifying hazardous substances according to their structure and establishing and utilizing a core model of a PBPK model for each family, in order to more quickly and efficiently evaluate human body exposure for each of these substances.

Disclosure

Technical Problem

One aspect provides a physiologically based pharmacokinetic (PBPK) core model that may be commonly applied to two or more compounds, wherein the core structure of the compounds includes the Formula 1 below.

Another aspect provides a method of providing information on external dose to a compound of a subject using the PBPK core model.

Another aspect provides a method of constructing a PBPK core model including constructing individual PBPK models of two or more compounds that has a common core structure; selecting compartments of the core model from the individual model structures; and constructing a PBPK core model using the compartments.

Technical Solution

One aspect provides a physiologically based pharmacokinetic (PBPK) core model that may be commonly applied to two or more compounds, wherein a core structure of the compounds includes the Formula 1 below.

The compound may be a perfluorinated chemical (PFC). In addition, the core structure of the compound may include one or more selected from the group consisting of Formula 1 to Formula 3 below.

[Formula 1]

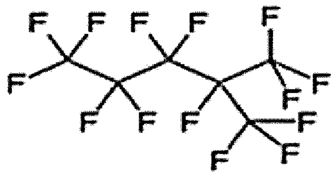

[Formula 2]

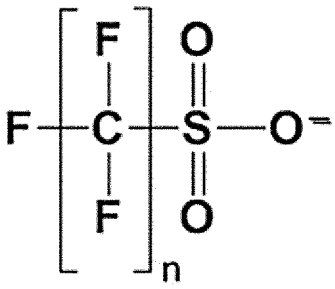

[Formula 3]

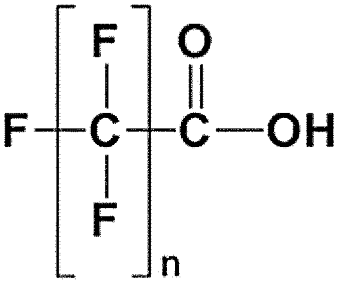

As used herein, the term "perfluoro/perfluorinated" refers to a compound in which all of the hydrogen atoms in the chain of the molecule have been replaced by fluorine atoms.

As used herein, the term "perfluorinated chemicals (PFCs)", also referred to as per- and polyfluoroalkyl substances (PFAS), refers to compounds in which all C-H in the chain have been replaced by C-F, and may include perfluorinated precursors that have the potential to be converted to perfluorinated compounds.

In an embodiment, the PFC may be one or more selected from the group consisting of compounds of the perfluorocarboxylic acids (PFCAs) family and compounds of the perfluoroalkane sulfonates (PFSAs) family.

In an embodiment, the PFSA family compounds may include perfluorooctanesulfonic acid (PFOS) family compounds.

In an embodiment, the PFCA family compounds may include perfluorooctanoic acid (PFOA) family compounds or perfluoropentanoic acid (PFPeA) family compounds.

As used herein, the term "PFSAs, or a compound of the PFCAs family" refers to PFSAs, PFCAs, salts thereof, derivatives thereof, and substances that may be PFSAs, or PFCAs.

The PFOS may be represented by the Formula 4 below. Wherein X below refers to an OH group or a salt form.

In an embodiment, the PFC may include a precursor or derivative of the PFC. Specifically, the PFC may include PFSAs, or substances that have the potential to degrade to PFCAs, or derivatives of PFSAs, or derivatives of PFCAs.

[Formula 4]

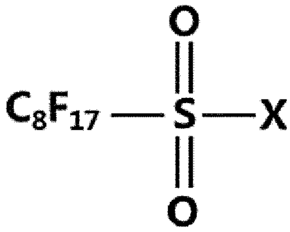

The PFOA may be represented by the Formula 5 below. X below refers to an OH group or a salt form.

[Formula 5]

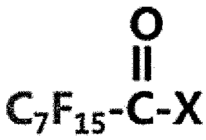

The "PFCA" may be those represented by the Formula 6 below. X below refers to an OH group or a salt form.

[Formula 6]

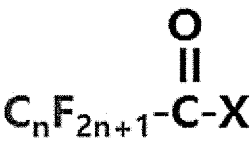

The "PFSA" may be those represented by the Formula 7. X below refers to an OH group or a salt form.

[Formula 7]

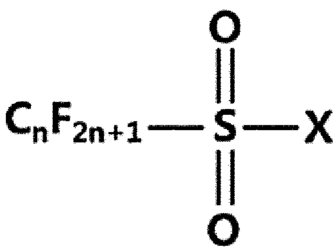

As used herein, the term "PBPK model (physiologically based pharmacokinetic model: PBPK model)" refers to a model in which each tissue and organ of a living organism is modeled in connection with the bloodstream in consideration of physiological characteristics in order to accurately predict the pharmacokinetics of a drug. Unlike conventional compartment models or pharmacokinetic models, the PBPK model may be applied to model physiological features, drug features, biological response features, metabolism, distribution characteristics, etc. Therefore, the PBPK model may predict the concentration of a drug or toxic substance in the blood and target organs under various conditions, and may be used to predict the concentration of a drug in the blood and target organs by developing a human PBPK model using PBPK models developed through various animal experiments and human physiological characteristics.

In the PBPK model, each tissue is represented by a compartment with intrinsic physiological (for example, blood flow, tissue or organ volume) and physicochemical (partition coefficient, etc.) parameter values. Organs and organ systems may be represented by boxes, and blood flow or material removal processes may be represented by arrows.

As used herein, the term "PBPK core model", also referred to as physiologically based pharmacokinetic (PBPK) core model, refers to a pharmacokinetic model that may be commonly applied to two or more compounds.

As used herein, the term "compartment" refers to the organization of the body into one or more compartments by grouping organs with similar distribution properties. The compartments may be used to calculate the time to concentration profile of drug. In addition, the compartment may be one that is generally assumed to be a reaction vessel that is homogeneously and completely mixed according to a well-stirred model. That is, the concentration of a substance within a tissue may be assumed to be the same and to have a specific distributional relationship to the venous blood concentration.

The compartments of the physiologically based pharmacokinetic model may include one or more selected from the group consisting of rich perfused tissue, slow perfused tissue, kidney, storage compartment, blood pool, gastrointestinal tract (G.I tract), and liver.

In an embodiment, the compartment may include a rich perfused tissue, a slow perfused tissue, a kidney, a storage compartment, a blood pool, a G.I tract, and a liver.

The PBPK core model may include one or more mass balance differential equations selected from the group consisting of Equation 1 to Equation 11 below.

[Equation 1]

$$V_{bl}\cdot\frac{dC_{bl}}{dt}=Free\cdot(Q_{ri}\frac{C_{ri}}{K_{ri}}+Q_{sl}\frac{C_{sl}}{K_{sl}}+Q_{li}\frac{C_{li}}{K_{li}}+\frac{T_mC_{ki}}{K_t+C_{ki}})$$
$$-Free\cdot(Q_{ri}+Q_{sl}+Q_{ki}+Q_{gi}+Q_{li})C_{bl}$$

[Equation 2]

$$\frac{dA_{oral}}{dt}=-K_{abs}A_{oral}$$

[Equation 3]

$$A_{oral}(t=0)=dose\cdot(1-F_u)$$

[Equation 4]

$$V_{gi}\frac{dC_{gi}}{dt}=Free\cdot(Q_{gi}C_{bl}-Q_{gi}\frac{C_{gi}}{K_{gi}}+Q_{li}\frac{C_{li}}{K_{li}}-K_f\frac{C_{gi}}{K_{gi}})+K_{abs}\cdot A_{oral}$$

[Equation 5]

$$V_{li}\frac{dC_{li}}{dt}=Free\cdot(Q_{li}C_{bl}+Q_{gi}\frac{C_{gi}}{K_{gi}}-Q_{li}\frac{C_{li}}{K_{li}}-Q_{li}\frac{C_{li}}{K_{li}})$$

[Equation 6]

$$V_{ki}\frac{dC_{ki}}{dt}=Free\cdot Q_{ki}C_{bl}+\frac{T_mC_{fil}}{K_t+C_{fil}}-Q_{ki}C_{ki}$$

[Equation 7]

$$V_{fil}\frac{dC_{fil}}{dt}=Free\cdot Q_{fil}C_{bl}-\frac{T_{m}C_{fil}}{K_{t}+C_{fil}}-K_{st}C_{fil}$$

[Equation 8]

$$\frac{dA_{st}}{dt}=K_{st}C_{fil}-K_{u}A_{st}$$

[Equation 9]

$$\frac{dA_{urine}}{dt}=K_{u}A_{st}$$

[Equation 10]

$$\frac{dA_{u}}{dt}=K_{u}A_{st}$$

[Equation 11]

$$\frac{dA_{f}}{dt}=K_{f}\cdot Q_{gi}\frac{C_{gi}}{K_{gi}}$$

Equation 1 represents the blood pool, Equation 2 and Equation 3 represent oral administration, Equation 4 represents rich perfused tissue, Equation 5 represents slow perfused tissue, Equation 6 represents the liver, Equation 7 represents the kidneys, Equation 8 represents the storage compartment, Equation 9 represents the G.I tract, Equation 10 represents the urine, and Equation 11 represents the feces, as differential equations.

As shown in FIG. 16 and Table 5 below, the K refers to a rate constant (1/hr), Q refers to a flow rate (L/hr), C refers to a concentration (mg/L or mmol/L), A refers to an amount (mg), Ku refers to a urine excretion rate constant (1/hr), and Kabs refers to an oral absorption rate constant (1/hr).

Of the subscripts, the he refers to heart, the rb refers to rest of body, the br refers to brain, the li refers to liver, the lu refers to lungs, the ki refers to kidneys, the gi refers to G.I tract, the fil refers to filtrate compartment, the oral refers to oral administration, the st refers to storage compartment, and the urine refers to urinary.

The parameter may be a physiological parameter, a partition coefficient, or a biochemical parameter. The physiological parameter may include an absorption rate, a metabolic parameter, a tissue diffusion constant, a protein binding, or a metabolism. In addition, the metabolic parameter may include a first order or second order rate constant, a maximum metabolic rate, or a Michaelis-Menten constant.

Another aspect provides a method of providing information on external dose to a compound of a subject using the PBPK core model.

The compound may be a PFC. In addition, the core structure of the compound may include one or more selected

from the group consisting of Formula 1 to Formula 3 below.

[Formula 1]

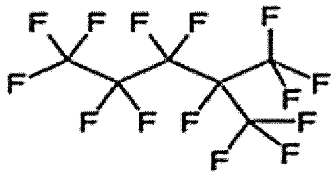

[Formula 2]

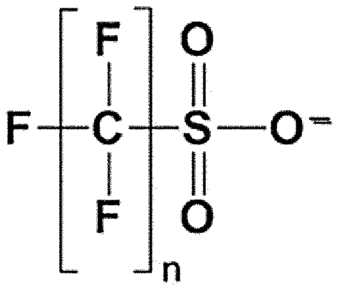

[Formula 3]

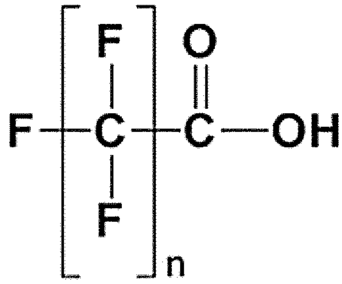

The PFC, exposure amount, core structure, and PBPK model are as described above.

The method of providing information on the exposure amount of the compound may include calculating a partition coefficient of the compound subject to exposure analysis;

calculating an internal dose of the compound subject to exposure analysis using the distribution coefficient and the PBPK model to calculate; and

comparing the internal dose to a blood concentration of the compound in the subject.

The partition coefficient may vary depending on the species, sex, body weight, etc. of the subject.

In addition, the method may include: 1) constructing a human PBPK model by replacing the animal physiological parameters (V or Q of Equation 1 to Equation 11) in the developed animal PBPK model with general human physiological parameter from Table 3 or Table 4, or by extrapolating the animal parameters from Table 6 to human parameters across species, etc.; 2) calculating an internal dose of the compound that is the subject of the exposure analysis using the human PBPK model; 3) comparing the internal dose to the blood concentration of the compound in the subject; and 4) calculating an external dose from human biomonitoring data (internal dose).

In exposure evaluation, the internal dose or the internal equivalent dose refers to the amount of a substance that penetrates an absorption barrier of an organism (for example, skin, lung tissue, gastrointestinal tract) and is absorbed through physical or biological processes, while external dose refers to the amount of a substance exposed to or ingested by a person or animal.

Another aspect provides a method of constructing a PBPK core model comrprises constructing individual PBPK

models of two or more compounds with a common core structure;

selecting a compartment of a core model through the individual model structure; and
Constructing a PBPK core model using the compartments.

The compound may be a PFC. In addition, the core structure of the compound may include one or more selected from the group consisting of Formula 1 to Formula 3 below.

[Formula 1]

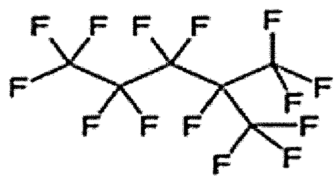

[Formula 2]

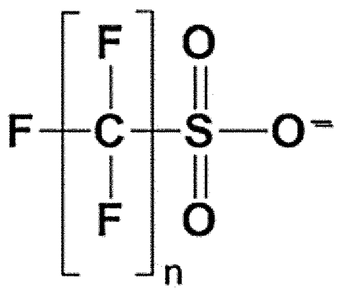

[Formula 3]

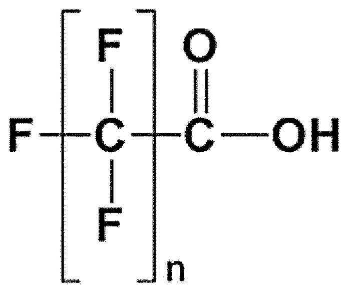

The PFC, exposure amount, core structure, and PBPK model are as described above.

The individual PBPK model may refer to a PBPK model of an individual compound included in the compound above and sharing a core structure. The individual compounds may be two or more, such as three or four.

In an embodiment, it has been confirmed that when two or more individual PBPK models are constructed, a core physiological model may be built that is generic to the compounds that share a core structure.

In the method, the process of building the individual PBPK models may be preceded by analyzing the characteristics of the compound. The compound may be predicted as a hazardous substance.

Analyzing the properties of the compound may refer to known literature or data of intravenous injection or oral administration of the compound to laboratory animals.

In the method, the process of building an individual PBPK model may include selecting a compound that is the subject of the model building; selecting two or more individual compounds that have a common core structure among the selected compounds; and building an individual PBPK model of the selected individual compound.

In the method, the process of selecting compartments of the core model may include the process of selecting tissues and organs with similar pharmacokinetic behavior as compartments of the core model by comparing the structure, pharmacokinetic profile, and absorption, distribution, metabolism, and excretion (ADME) characteristics of the two or

more individual PBPK models.

The selection may be by using curve fitting.

The compartment may include one or more selected from the group consisting of a rich perfused tissue, a slow perfused tissue, a kidney, a storage compartment, a blood pool, a G.I tract, and a liver.

In an embodiment, the compartment may include a rich perfused tissue, a slow perfused tissue, a kidney, a storage compartment, a blood pool, a G.I tract, and a liver.

The method may additionally include, calculating a physicochemical parameter, physiological parameter, or biochemical parameter of the compound to be measured for exposure; and establishing a mass balance differential equation of the selected compartment.

The physicochemical parameter may include a partition coefficient.

The physiological parameter may include a blood flow or a tissue volume. The blood flow of the tissue or the volume of the tissue may be values from known literature.

The biochemical parameter may include protein binding rate and Km value.

The calculations may be based on parameters from known literature, or may be based on values obtained by injecting the hazardous substance into the animal, or may be based on values obtained by curve fitting.

The method may additionally include, curve fitting the predicted time-blood concentration of the hazardous substance from the constructed PBPK core model and the experimental value of time-blood concentration of the hazardous substance obtained from animal experiments; and evaluating the constructed PBPK core model.

The process of evaluating may include the process of verification and validation of the model.

The verification and validation may include evaluating the parameters and the differential equations. Specifically, it may be to evaluate whether the shape of the curve matches the experimental value and, for example, whether the residuals plot for the difference between the measured value and the predicted value is evenly distributed and not skewed in either direction around the zero value.

In an embodiment, the method may evaluate that the derived model is valid if the shape of the derived curve tends to match the experimental value or is evenly distributed.

The method may additionally include the process of deriving a mass balance differential equation of the compartment. The mass balance differential equation may include one or more selected from the group consisting of Equation 1 to Equation 11.

The mass balance differential equation may include parameter values, such as blood flow, organ volume, or partition coefficient. The parameters may be derived using values known in the art or through additional experiments.

The method may include the process of performing curve fitting of the parameters of the derived PBPK core model. For example, the method may include the process of curve-fitting the PBPK core model with the pharmacokinetic profile of the target compound to derive final parameters. The derived final parameters may additionally include altering the axis scaling, etc.

In the method, the process of building a PBPK core model using the compartments may include the process of deriving a structure of the PBPK core model using the mass balance differential equation or final parameters of the compartments.

The deriving method may include a method of coding a mass balance differential equation and parameter.

The method may additionally include the process of subsequently performing a risk evaluation of the compound with the derived PBPK core model.

In another aspect, the method may include providing a PBPK core model derived by the method, wherein the model is used for risk evaluation of a compound.

Advantageous Effects

One aspect provides, a physiologically based pharmacokinetic (PBPK) core model which may be commonly applied to two or more compounds that share the same core structure, and a method of constructing the PBPK core model. According to the PBPK core model and the method of constructing the PBPK core model, it is possible to establish a PBPK model simply by deriving a partition coefficient without constructing a separate PBPK model for each individual compound, and there is an effect that the exposure test of the hazardous substance to be evaluated can be efficiently performed.

Description of Drawings

FIG. 1 is an image classifying a perfluorinated chemicals (PFCs) into the perfluorooctanesulfonic acid (PFOS) family, perfluorooctanoic acid (PFOA) family, polyfluoroalkyl substances (PFAS) family, and perfluorocarboxylic acid (PFCA)

family.

FIG. 2 is an image illustrating the structure of a physiologically based pharmacokinetic (PBPK) model of PFHxS in rats.

FIG. 3 is an image of coding of differential equations of an individual PBPK model of PFHxS.

FIG. 4 is an image illustrating a parameter input screen of an individual PBPK model of PFHxS.

FIG. 5 is an image illustrating a time-blood concentration graph for each compartment of an individual PBPK model of PFHxS.

FIG. 6 is an image illustrating a graph upon completion of curve fitting of an individual PBPK model of PFHxS.

FIG. 7 is an image illustrating differential equation coding and a parameter input screen of an individual PBPK model of PFOA.

FIG. 8 is an image illustrating the structure of an individual PBPK model for PFOA.

FIG. 9 is an image illustrating differential equation coding and a parameter input screen of an individual PBPK model of PFOS.

FIG. 10 is an image illustrating the structure of an individual PBPK model for PFOS.

FIG. 11 is an image illustrating a process of selecting a compartment to derive a PBPK core model from individual PBPK models.

FIG. 12 is a graph illustrating the structure of a PBPK core model of PFC.

FIG. 13 is an image of establishing an analysis method for a biological sample after single-dose administration and oral administration of PFHxS in rats.

FIG. 14 is a graph illustrating a calibration curve, accuracy and precision of the analysis method after single-dose administration and oral administration of PFHxS in rats.

FIG. 15 is a graph illustrating the pharmacokinetics of PFHxS after oral administration in rats.

FIG. 16 is an image illustrating the final parameters from interspecies extrapolation of a PBPK core model.

FIG. 17 is a graph illustrating the correlation between average blood concentration and external dose according to repeated PFHxS exposure.

FIG. 18 is a schematic diagram illustrating a process of calculating a PFHxS standard exposure amount.

FIG. 19 is a schematic diagram illustrating a process of calculating a margin of safety (MoS) for PFHxS.

FIG. 20 is a graph comparing the internal dose of PFHxS and the blood concentration of PFHxS in the human body.

FIG. 21 is a schematic diagram illustrating a method of one aspect of calculating a core physiological model.

Mode for Invention

Preferred embodiments of the present disclosure are described below to facilitate an understanding of the present disclosure. However, the following embodiments are provided only to facilitate understanding of the present disclosure and are not intended to limit the scope of the present disclosure.

**[Preparation Example]**

**Preparation Example 1. Selection of hazardous substance to be analyzed and investigation of pharmacokinetics**

To derive the physiologically-based pharmacokinetics (PBPK) core model, the hazardous substances to be analyzed were selected. First, it was confirmed whether the biomonitoring results of candidate hazardous substances were currently available.

Specifically, as shown in Table 1 below, a list of substances for which biomonitoring results are currently available was provided by the Ministry of Food and Drug Safety (hereinafter referred to as MFDS), and each substance was classified according to similarity and family of molecular structures. As a result of the classification, a total of 118 hazardous substances were classified into 11 families.

[Table 1]

| Reported biomonitoring data from 2006-2013 by MFDS | |
|---|---|
| Family | Number |
| Metals | 4 types |
| Paraben | 4 types |
| Phenols | 3 types |
| Zearalenone | 2 types |

(continued)

| Reported biomonitoring data from 2006-2013 by MFDS | | | |
|---|---|---|---|
| Af-lysine | | 1 type | |
| Acrlyamide | | 3 types | |
| Heterocyclinc amines | | 1 type | |
| Dioxins | | 3 types | |
| PFCs | | 19 types | |
| Phthalates | | 18 types | |
| POPs | PBEDEs | 12 types | 52 types |
| | PCBs | 15 types | |
| | OCPs | 25 types | |
| 11 families (8 others) | | 118 types | |

Based on the molecular structure, various hazardous substances may be classified into families, and in particular, if they have a common core structure, they may be classified into the same family.

FIG. 1 is an image classifying a perfluorinated chemical (PFC) into perfluorooctanesulfonic acid (PFOS) family, perfluorooctanoic acid (PFOA) family, polyfluoroalkyl substances (PFAS) family, and perfluorocarboxylic acid (PFCA) family.

Table 2 summarizes the core structurestructure of PFCA and the hazardous substance that has the core structure.

[Table 2]

| Core structure | |
|---|---|
| 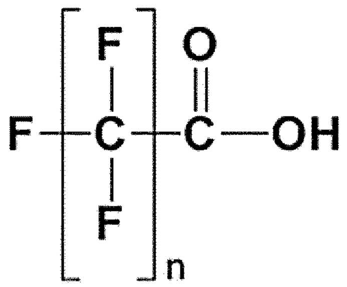 | Perfluorocarboxylic acids (PFCAs) |
| List of hazardous substances | |
| 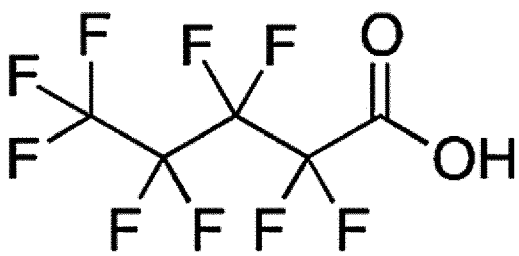 | Perfluoropentanoic acid (PFPeA) |
| 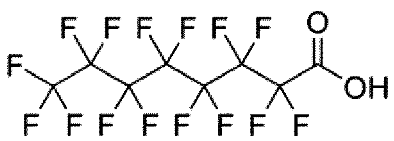 | Perfluorooctanoic acid (PFOA) |

(continued)

| List of hazardous substances | |
|---|---|
| 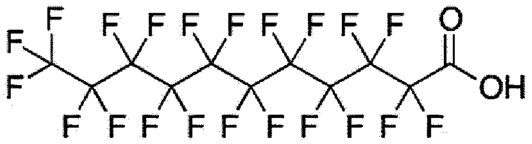 | Perfluoroundecanoic acid (PFUnDA) |
| PFCs family, 3 of 19 total hazardous substances | |

Next, a pharmacokinetics of hazardous substance were investigated using previously reported literature, etc. In particular, data on blood concentrations, tissue distributions, and excretions were obtained. Animal experiments were conducted on rats for the unobtainable data.

**Preparation Example 2. Evaluating pharmacokinetics of hazardous substance in laboratory animal**

**2.1 Evaluation after the single-dose intravenous injection of a hazardous substance in laboratory animal**

To determine plasma concentration-time curves, pharmacokinetics parameters, and urinary and fecal excretion after the intravenous injection in laboratory animals, small rodents such as mice and rats at 8 weeks of age (240 g to 260 g) were anesthetized by appropriate methods and a PE-50 tube was inserted into the femoral vein. Specifically, the intravenous injection of hazardous substances was performed through the femoral vein or tail vein. Injection needles of 27 G or less for mice and 19 G or less for rats were used.

Sample collection was carried out in the case of rats after administration of hazardous substances and transferred to a metabolic cage, and blood was drawn through the jugular vein at given times based on the time of administration according to the predesigned animal experiment design. At each sampling time, 0.3 mL of blood was drawn and filled into a polyethylene tube with heparin sodium (100 I.U / mL) to prevent blood clotting. Finally, the collected blood was centrifuged at 10,000 g for 10 minutes and the plasma was collected and stored at -70 °C until analysis. After the last blood collection time point, the rats were bled to death, and each organ was harvested and washed with phosphate-buffered saline to remove blood from the organ surface. Next, the organs were stored at -70°C until analysis. In addition, urine and feces were collected at regular intervals from the time of administration and stored at -70°C until analysis.

**2.2 Evaluation after the single-dose oral administration of a hazardous substance in laboratory animal**

To determine plasma concentration-time curves, pharmacokinetics parameters, and urinary and fecal excretion during oral administration in experimental animals, rodents such as mice and rats at 8 weeks of age (240 g to 260 g) were fasted for 10 hours or more before the experiment. The fasting period was limited to 18 hours, as too long a fasting period may affect the physiological functions of the animals, and water was allowed to be consumed freely during the fasting period.

For the preparation of the test substance, the dosage for rats was about 1.0 mL for 200 g rats, and for mice, the dosage was about 0.2 mL for 20 g mice. In addition, the test substance was

Samples were collected, by venipuncture and centrifuged at 10000 g to separate plasma, urine and feces were collected. After collection, samples were stored at -70°C until analysis.

**2.3 Evaluation after intravenous infusion of a hazardous substance in laboratory animal**

In order to measure steady-state plasma and tissue concentration and blood-tissue partition coefficient ($K_p$) in a laboratory animal, small rodents such as mice and rats were anesthetized by appropriate methods at 8 weeks of age (240 g to 260 g), and administered the hazardous substance intravenously.

Samples were collected by venipuncture and centrifuged at 10000 g to separate plasma, and tissues were homogenized in phosphate-buffered saline. The collected plasma and tissues were stored at -70 °C until analysis.

**2.4 Data processing**

The calculation of non-compartmental pharmacokinetics parameters from the data obtained in Example 2.1 to Example 2.3 was performed using WinNonlin software (Certara, NJ, USA). Verification of Statistical analysis was performed using SPSS (SPSS Inc. USA).

**Preparation Example 3. Preparation of parameters and partition coefficients**

**3.1 General parameter values**

In the PBPK model, values for ventilation rate, cardiac output, tissue blood flow, and tissue weight are specified for the individual animal or human being simulated, and the values of these parameters are body weight-dependent.

Table 3 and Table 4 below show general parameter values used in the PBPK model of the present disclosure.

[Table 3]

| **Physiological Parameters** | **mouse** | **Rat** | **Human** |
|---|---|---|---|
| Body weight (BW) (kg) | 0.025 | 0.25 | 70 |
| Tissue volume (fraction of BW) | | | |
| Liver | 0.055 | 0.04 | 0.026 |
| Fat | 0.1 | 0.07 | 0.19 |
| Organ | 0.05 | 0.05 | 0.05 |
| Muscle and skin | 0.7 | 0.75 | 0.62 |
| Cardiac output (Qc) (L/min) | 0.017 | 0.083 | 6.2 |
| Tissue perfusion (fraction of Qc) | | | |
| Liver | 0.25 | 0.25 | 0.26 |
| Fat | 0.09 | 0.09 | 0.05 |
| Organs | 0.51 | 0.51 | 0.44 |
| Muscle and skin | 0.15 | 0.15 | 0.25 |
| Minute volume (L/min) | 0.037 | 0.174 | 7.5 |
| Alveolar ventilation (L/min) | 0.025 | 0.117 | 5 |

[Table 4]

| Tissue | Rat | | Human | |
|---|---|---|---|---|
| | Tissue volume (mL) | Blood flow (mL/h) | Tissue volume (mL) | Blood flow (mL/h) |
| Blood | 20.4 | 2580 | 5400 | 379800 |
| Heart | 0.8 | 234 | 300 | 14400 |
| Brain | 0.7 | 18 | 1500 | 42000 |
| Lung | 1.0 | 2580 | 1200 | 379800 |
| Adipose tissue | 10.0 | 24 | 12200 | 15600 |
| Muscle | 122.0 | 450 | 30000 | 45000 |
| Kidney | 1.8 | 520 | 300 | 74400 |
| G.I tract | 10.0 | 450 | 2400 | 66000 |
| Liver | 8.0 | 828 | 1500 | 87000 |
| Spleen | 1.3 | 36 | 192 | 4620 |

**3.2 Calculating biochemical parameters**

The types of physiological parameters used in the physiologically based pharmacokinetic core model include absorption rates, metabolic parameters, tissue distribution constants, serum protein binding, and metabolic constants, etc. Metabolic parameters refer to 1st order or 2nd order rate constants, maximum metabolic rate, Michaelis-Menten

constant, etc.

In the case of absorption rate, metabolic parameters, and tissue distribution constants, a PBPK model was derived and then calculated by fitting to data obtained in vivo. In this case, the pharmacokinetic data were obtained after intravenous infusion of the drug into the animal.

### 3.3 Calculation of partition coefficient

To derive the PBPK model, the partition coefficient was calculated by obtaining steady-state in vivo data by performing an actual animal experiment as shown in Example 2 above.

**[Example]**

**Example 1.** Method of building physiologically based pharmacokinetic (PBPK) core model of perfluorinated chemical (PFC)

### 1.1 Construction of PFOS physiological pharmacokinetic model

In order to derive a PBPK core model that may be commonly applied to a PFC, individual PBPK models of PFHxS, PFOA, and PFOS, a type of PFC, were constructed in rats.

### 1.1.1. PBPK model compartment selection and structure derivation of PFHxS

First, a compartment of a PBPK model were selected by considering the toxicity, pharmacokinetic profile, absorption, distribution, metabolism, and excretion (ADME) characteristics, and excretion mechanism of PFOS through literature review and previous pharmacokinetic studies.

As a result, brain, heart, lung, rest of body (spleen, muscle, adipose tissue), kidney, liver, and gastrointestinal tract (G.I tract) were selected as compartments, and filtrate compartment and storage compartment were added to consider the mechanism of excretion from the kidneys.

FIG. 2 is an image illustrating the structure of the PBPK model of PFHxS in rats.

As a result, as shown in FIG. 2, the PBPK model structure of PFOS could be derived.

### 1.1.2. Derivation and coding of PBPK model differential equation of PFHxS

Appropriate differential equations were established for the compartments and structures derived in Example 1.1.1 above. As a result, the differential equations of Equation A to Equation M below were derived.

[Equation A]

$$V_{bl}\frac{dC_{bl}}{dt} = Free \cdot (Q_{he}\frac{C_{he}}{K_{he}} + Q_{rb}\frac{C_{rb}}{K_{rb}} + Q_{br}\frac{C_{br}}{K_{br}} + Q_{li}\frac{C_{li}}{K_{li}} + Q_{lu}\frac{C_{lu}}{K_{lu}} + Q_{ki}\frac{C_{ki}}{K_{ki}})$$

$$-Free \cdot (Q_{he} + Q_{rb} + Q_{br} + Q_{li} + Q_{lu} + Q_{ki} + Q_{gi} + Q_{fil})C_{bl}$$

[Equation B]

$$\frac{dA_{oral}}{dt} = -K_{abs}A_{oral}$$

[Equation C]

$$A_{oral}(t=0) = dose \cdot (1 - F_u)$$

[Equation D]

$$V_{gi}\frac{dC_{gi}}{dt}=Free\cdot(Q_{gi}C_{bl}-Q_{gi}\frac{C_{gi}}{K_{gi}}+Q_{li}\frac{C_{li}}{K_{li}}-K_{f}\frac{C_{gi}}{K_{gi}})+K_{abs}\cdot A_{oral}$$

[Equation E]

$$V_{li}\frac{dC_{li}}{dt}=Free\cdot(Q_{li}C_{bl}+Q_{gi}\frac{C_{gi}}{K_{gi}}-Q_{li}\frac{C_{li}}{K_{li}}-Q_{li}\frac{C_{li}}{K_{li}})$$

[Equation F]

$$V_{ki}\frac{dC_{ki}}{dt}=Free\cdot Q_{ki}C_{bl}+\frac{T_{m}C_{fil}}{K_{t}+C_{fil}}-Q_{ki}C_{ki}$$

[Equation G]

$$V_{fil}\frac{dC_{fil}}{dt}=Free\cdot Q_{fil}C_{bl}-\frac{T_{m}C_{fil}}{K_{t}+C_{fil}}-K_{st}C_{fil}$$

[Equation H]

$$\frac{dA_{st}}{dt}=K_{st}C_{fil}-K_{u}A_{st}$$

[Equation I]

$$\frac{dA_{urine}}{dt}=K_{u}A_{st}$$

[Equation J]

$$V_{lu}\frac{dC_{lu}}{dt}=Free\cdot(Q_{lu}C_{bl}-Q_{lu}\frac{C_{lu}}{K_{lu}})$$

[Equation K]

$$V_{he}\frac{dC_{he}}{dt}=Free\cdot(Q_{he}C_{bl}-Q_{he}\frac{C_{he}}{K_{he}})$$

[Equation L]

$$V_{br}\frac{dC_{br}}{dt}=Free\cdot(Q_{br}C_{bl}-Q_{br}\frac{C_{br}}{K_{br}})$$

[Equation M]

$$V_{rb}\frac{dC_{rb}}{dt}=Free\cdot(Q_{rb}C_{rb}-Q_{rb}\frac{C_{rb}}{K_{rb}})$$

Equation A corresponds to a differential equation for the blood pool (bl), Equation B and Equation C for oral administration, Equation D for the G.I tract, Equation E for the liver, Equation F for the kidneys, Equation G for the filtrate compartment, Equation H and Equation I for the storage compartment, Equation J for the lung, Equation K for the kidneys, Equation L for the brain, and Equation M for the rest of the body.

As shown in FIG. 16 and Table 5 below, K refers to a rate constant (1/hr), Q refers to a flow rate (L/hr), C refers to a concentration (mg/L or mmol/L), A refers to an amount (mg), Ku refers to a urine excretion rate constant (1/hr), and Kabs refers to an oral absorption rate constant (1/hr).

Of the subscripts, the he refers to heart, the rb refers to rest of body, the br refers to brain, the li refers to liver, the lu refers to lungs, the ki refers to kidneys, the gi refers to G.I tract, the fil refers to filtrate compartment, the oral refers to oral administration, the st refers to storage compartment, and the urine refers to urinary.

Table 5 is a table showing the definition of each variable of the above Equation A to Equation M.

[Table 5]

| | |
|---|---|
| | $Q$ = flow rate ($L/hr^{-1}$) |
| $A$ = amount (mg) | $r$ = richly perfused tissues |
| $a$ = arterial blood | $s$ = slowly perfused tissues |
| $C_{alv}$ = alveolar air | $t$ = tissue:blood |
| $b$ = blood-air | $V$ = volume (L) |
| $C$ = concentration (mg/L or mmol/L) | $v$ = mixed venous blood |
| $c$ = cardiac output | $V_f$ = venous blood leaving fat |
| $f$ = fat | $V_l$ = venous blood leaving liver |
| $\in h$ = inhaled air | $V_{max}$ = maximal velocity of enzymatic reaction ($mg/hr^{-1}$) |
| $K_m$ = Michaelis-Menten affinity constant (mg/L) | $V_r$ = venous blood leaving richly perfused tissue |
| $l$ = liver | |
| $P$ = partition coefficient | $V_s$ = venous blood leaving poorly perfused tissue |
| $p$ = pulmonary ventilation | |

Next, the above differential equations were coded using the Berkeley Madonna program.

FIG. 3 is an image of coding the differential equations of the individual PBPK model of PFHxS.

FIG. 4 is an image illustrating the parameter input screen of the individual PBPK model of PFHxS.

As shown in FIG. 3 and FIG. 4, the initial values were input, and the differential equations represented by Equation 1 to Equation 11 were coded. In particular, the parameters used for coding include blood flow, organ volume, and partition coefficient, etc. and the values of the above parameters were obtained through literature review and previous experiments. In the case of the predicted parameters, the initial values were arbitrarily assigned and later calculated by curve fitting.

FIG. 5 is an image illustrating a time-blood concentration graph for each compartment of an individual PBPK model of PFHxS.

As a result of executing the coding as described above, as shown in FIG. 5, a time-blood concentration graph for each compartment could be confirmed.

### 1.1.1. Execution of curve fitting

Curve fitting was performed with a PBPK model derived in the above example using data from the pharmacokinetic study in rats for PFOS. The curve fitting was performed using the Berkeley Madonna program. Specifically, clicking the curve fit button in the parameters section of the menu window, then clicking the parameter to predict by fitting all parameters included in the differential equation, and then clicking "Add". In addition, specify the compartment of the selected dataset to perform curve fitting and set a data corresponding to the same compartment in "To Dataset". After the above settings were completed, curve fitting was performed by pressing OK.

FIG. 6 is an image illustrating the curve fitting graph of individual PBPK models of PFHxS.

As a result, as shown in FIG. 6, it was possible to confirm a fitted prediction curve after curve fitting was completed.

### 1.2 PBPK model construction of PFOA

In the same manner as in Example 1.1, a PBPK model of PFOA was derived.
FIG. 7 is an image illustrating of the differential equation coding and parameter input screen of the individual PBPK model of PFOA.
FIG. 8 is an image illustrating the structure of the individual PBPK model for PFOA.
Specifically, physiological parameters and partition coefficients, etc., as shown in FIG. 7 were used, and as a result, the PBPK model structure of PFOA was derived as shown in FIG. 8.

### 1.3 PBPK model construction of PFOS

In the same manner as in Example 1.1, a PBPK model of PFOS was derived.
FIG. 9 is an image illustrating of the differential equation coding and parameter input screen of the individual PBPK model of PFOS.
FIG. 10 is an image illustrating the structure of the individual PBPK model for PFOS.
Specifically, physiological parameters and partition coefficients, etc., as shown in FIG. 9 were used, and as a result, the PBPK model structure of PFOS was derived as shown in FIG. 10.

### 1.4 Construction of PBPK core model from individual PBPK model

#### 1.4.1. Construction of PBPK core model

Similarities in ADME characteristics were identified through literature review and previous pharmacokinetic studies of at least three compounds among the selected compounds in the same family.
FIG. 21 is a schematic illustration of one aspect of a method of generating a core physiologic model.
Specifically, the comparison of the ADME characteristics of PFOA, PFOS, and PFHxS shown in Example 1.1 to Example 1.3 above and a comparison of individual PBPK models, a PBPK core model for the entire PFC was derived, as shown in FIG. 21.

#### 1.4.2. Calculation of physiological parameter and intrinsic parameter of PBPK core model

The physiological parameters (organ volume and blood flow velocity) required for a PBPK core model were taken from numerical values reported in the literature. In addition, parameters to describe the pharmacokinetic characteristics of each hazardous substance family were calculated by curve fitting.

#### 1.4.3. Deriving the structure of PBPK core model

FIG. 11 is an image illustrating the process of selecting a compartment to derive a PBPK core model from the individual PBPK models.
As shown in FIG. 11, tissues and organs with similar pharmacokinetic behavior were selected as compartments for the core model by comparing the individual model structures, pharmacokinetic profiles, and ADME characteristics of PFOA, PFOS, and PFHxS.
As a result, the final compartments of the PBPK core model were selected as rich perfused tissue, slow perfused tissue, kidney, storage compartment, blood pool, G.I tract, and liver.
Specifically, the GI tract was chosen because it is the primary absorption site for PFCs from external exposure, and the liver is the organ where PFCs accumulate the most. Kidney was selected to illustrate the behavior of PFCs in excretion, while other tissues and organs were grouped into rich/slow-perfused tissues based on blood flow.
FIG. 12 is a graph illustrating the PBPK core model structure of PFCs.
As a result, as shown in FIG. 12, the structure of the PBPK core model of PFC was derived.

#### 1.4.4. Derivation and coding of PBPK core model differential equation

The appropriate differential equation for the above derived compartments and structures were established. As a result, the differential equations of Equation 1 to Equation 11 below were derived.

[Equation 1]

$$V_{bl} \cdot \frac{dC_{bl}}{dt} = Free \cdot (Q_{ri}\frac{C_{ri}}{K_{ri}} + Q_{sl}\frac{C_{sl}}{K_{sl}} + Q_{li}\frac{C_{li}}{K_{li}} + \frac{T_m C_{ki}}{K_t + C_{ki}})$$
$$- Free \cdot (Q_{ri} + Q_{sl} + Q_{ki} + Q_{gi} + Q_{li})C_{bl}$$

[Equation 2]

$$\frac{dA_{oral}}{dt} = -K_{abs}A_{oral}$$

[Equation 3]

$$A_{oral}(t=0) = dose \cdot (1 - F_u)$$

[Equation 4]

$$V_{gi}\frac{dC_{gi}}{dt} = Free \cdot (Q_{gi}C_{bl} - Q_{gi}\frac{C_{gi}}{K_{gi}} + Q_{li}\frac{C_{li}}{K_{li}} - K_f\frac{C_{gi}}{K_{gi}}) + K_{abs} \cdot A_{oral}$$

[Equation 5]

$$V_{li}\frac{dC_{li}}{dt} = Free \cdot (Q_{li}C_{bl} + Q_{gi}\frac{C_{gi}}{K_{gi}} - Q_{li}\frac{C_{li}}{K_{li}} - Q_{li}\frac{C_{li}}{K_{li}})$$

[Equation 6]

$$V_{ki}\frac{dC_{ki}}{dt} = Free \cdot Q_{ki}C_{bl} + \frac{T_m C_{fil}}{K_t + C_{fil}} - Q_{ki}C_{ki}$$

[Equation 7]

$$V_{fil}\frac{dC_{fil}}{dt} = Free \cdot Q_{fil}C_{bl} - \frac{T_m C_{fil}}{K_t + C_{fil}} - K_{st}C_{fil}$$

[Equation 8]

$$\frac{dA_{st}}{dt} = K_{st}C_{fil} - K_u A_{st}$$

[Equation 9]

$$\frac{dA_{urine}}{dt} = K_u A_{st}$$

[Equation 10]

$$\frac{dA_u}{dt} = K_u A_{st}$$

[Equation 11]

$$\frac{dA_f}{dt} = K_f \cdot Q_{gi} \frac{C_{gi}}{K_{gi}}$$

Equation 1 represents the blood pool, Equation 2 and Equation 3 represent oral administration, Equation 4 represents rich perfused tissue, Equation 5 represents slow perfused tissue, Equation 6 represents the liver, Equation 7 represents the kidneys, Equation 8 represents the storage compartment, Equation 9 represents the G.I tract, Equation 10 represents the urine, and Equation 11 represents the feces, as differential equations.

As shown in FIG. 16 and Table 5, K refers to a rate constant (1/hr), Q refers to a flow rate (L/hr), C refers to a concentration (mg/L or mmol/L), A refers to an amount (mg), Ku refers to a urine excretion rate constant (1/hr), and Kabs refers to an oral absorption rate constant (1/hr).

Of the subscripts, the he refers to heart, the rb refers to rest of body, the br refers to brain, the li refers to liver, the lu refers to lungs, the ki refers to kidneys, the gi refers to G.I tract, the fil refers to filtrate compartment, the oral refers to oral administration, the st refers to storage compartment, and the urine refers to urinary.

As described above, the mass balance differential equations for the compartments were established and coded using the Berkeley Madonna program. The coding method is the same as described above.

In addition, although the structure and formula of the PBPK core model of the same family of hazardous substances are the same, the partition coefficients have different values due to the unique physicochemical properties of the substances. Therefore, in order to apply the PBPK core model, minimal experiments were required, such as calculating the partition coefficient.

**Example 2. Validation of perfluorinated chemical (PFC) physiologically based pharmacokinetic (PBPK) core model**

**2.1 Investigation of toxicity and pharmacokinetics of PFHxS**

FIG. 13 is an image of established an analysis method in biological samples after a single dose oral administration of PFHxS in rats.

As shown in FIG. 13, an animal experiment plan was conducted to evaluate the pharmacokinetics of PFHxS. As shown in FIG. 14, the pharmacokinetics and tissue distribution of PFHxS were evaluated in blood, tissue, urine, and fecal samples obtained after single dose Intravenous or oral administration to SD rats.

**2.2 Extrapolation process using PBPK core model**

Since the PBPK core model based on rats has limitations in human body application and the collection of human body data has limitations, extrapolation was performed using in vitro and in vivo data.

**2.2.1. Performing interspecies extrapolation**

In order to extrapolate the PBPK core model based on rats from animals to humans, quantitative values of parameters such as partition coefficients, metabolic rate constants, etc. were required. Therefore, the parameters were converted.

For the conversion, some parameters were taken from literatures whereas others were derived by allometric scaling. Gender was considered when there were difference in physiological parameters or partition coefficients. Table 6 below shows the scaling equations for the parameters.

[Table 6]

| Parameter [Unit] | B | Equation |
|---|---|---|
| Volume [L or kg] | 1 | VF= VFc x BW |
| Flows [L/hr] | 0.75 | QC= QCcXBW0.75QP= QPc x BW0.75 |
| Clearance [L/hr] | 0.75 | Vmax= VmaxcXBW0.75 |
| Rate constants [/hr] | -0.25 | KF= KFc/BW-0.25 |
| Y=a*Xb (Y: PBPK parameters, X: body weight), where a is the allometric coefficient and b is the allometric exponent. | | |

In Table 6 above, the meaning of each variable is as follows: BW = body weight (kg), VF = volume of organ/tissue (L or kg), VFc = volume of organ/tissue per kg body weight (L/kg), QC = cardiac output (1/hr), QCc = cardiac output per kg body weight (1/hr/kg), Qp = blood flow in organ/tissue as percentage of cardiac output (%), QPc = blood flow in organ/tissue as percentage of cardiac output per kg body weight (%/kg), Vmax = maximum rate for metabolism (μmol/hr), Vmaxc = maximum rate for metabolism per kg body weight (μmol/hr/kg), KF = 1st order elimination constant (1/hr), KFc = 1st order elimination constant per body weight (1/hr/kg).

### 2.2.2. Performing a duration adjustment and dose adjustment

Duration adjustments were made to have the same dose metric. Specifically, if the appropriate dose metric for the target substance is the blood concentration area under the curve (AUC), the exposure duration was first determined in the PBPK model, and the continuous exposure concentration corresponding to the same AUC was determined through repeated simulations of the model.

## 2.3 Risk assessment using PBPK core model

### 2.3.1. Collection of PFHxS pharmacokinetic data

In order to evaluate the risk of PFHxS among PFCs, PFHxS pharmacokinetic data were collected as described above.

FIG. 13 is an image of established analysis method in a biological sample after a single dose and oral administration of PFHxS in rats.

FIG. 14 is a graph illustrating the calibration curve, accuracy and precision of the analysis method after a single dose and oral administration of PFHxS in rats.

Next, as shown in FIG. 13, the bioanalysis method for PFHxS was established. In addition, as shown in FIG. 14, the bioanalysis method was validated using calibration curves and accuracy/precision results..

After establishing the analysis method as described above, a plan was established for animal experiments. Specifically, rats were administered a single intravenous or oral dose of PFHxS, and then blood, tissue, urine, and fecal samples were collected to evaluate the pharmacokinetics and tissue distribution of PFHxS.

FIG. 15 is a graph illustrating the animal study results after oral administration of PFHxS in rats.

As a result, as shown in FIG. 15, a blood concentration-time curve and a graph of the cumulative amount in feces and urine were obtained after oral administration of PFHxS in rats.

### 2.3.2. Derivation of final parameter

Next, the results of the evaluation of PFHxS pharmacokinetics and tissue distribution in rats as described above were extended and applied to the PBPK core model shown in FIG. 12. Specifically, the parameters obtained from the pharmacokinetics and tissue distribution of PFHxS were applied to the model and curve fitting was performed to confirm that the results were well applied to the PBPK core model. At the same time, other physiological parameters were optimized to ensure that the blood concentration predictions obtained by curve fitting were in good agreement with the experimental values.

FIG. 16 is an image illustrating the final parameter according to interspecies extrapolation of the PBPK core model.

As a result, as shown in FIG. 16, the final parameter were derived interspecies extrapolation and estimation.

### 2.2.3. Calculation of correlation between mean blood concentration and external dose

The final parameters were used to calculate the correlation between the mean blood concentration at steady state and the external dose during repeated exposure to PFHxS. Specifically, using a human PBPK model constructed using human physiological parameters and substance-intrinsic parameters, a dose range was simulated and set, and the correlations described above were derived using regression analysis.

FIG. 17 is a graph illustrating the correlation between the mean blood concentration following repeated exposure to PFHxS and external dose.

As a result, as shown in FIG. 17, a graph of the correlation between the mean blood concentration and the external dose according to repeated exposure to PFHxS was derived.

### 2.2.4. PFHxS exposure calculation and risk assessment

Next, the interspecies uncertainty factor (UFA) was applied to calculate the steady-state $C_{average,human}$ in humans. Specifically, the PFHxS $POD_{human}$ in the human body was calculated by using the correlation equation between the average blood concentration (Caverage) and the external dose during repeated exposure to PFHxS from which $C_{average,human}$ was derived. Next, the intraspecies uncertainty factor (UFH) was applied to the $POD_{human}$ to finalize the PFHxS standard exposure in the human body.

FIG. 18 is a schematic diagram illustrating a process of calculating a PFHxS standard exposure amount.

As a result, as shown in FIG. 18, the standard exposure amount of PFHxS in the human body was 9.70 μg/kg/day.

### 2.2.5. PFHxS exposure calculation and risk assessment

The PFHxS reference standard dose for external dose to PFHxS was used to derive the margin of safety (MoS) of PFHxS for men and women. Specifically, the blood PFHxS concentrations of men in Siheung (2.29 ng/mL) and women in Daegu (1.75 ng/mL), which are highly exposed to PFHxS, were substituted into the above correlation equation between the average blood concentration (Caverage) and the external dose to calculate the external dose.

FIG. 19 is a schematic diagram illustrating the process of calculating the MoS for PFHxS.

As a result, as shown in FIG. 19, the MoS of PFHxS using the PFHxS standard exposure (9.7 μg/kg/day) was derived as 180.3 and 235.9 for men and women, respectively.

### 2.2.6 Comparison of PFHxS internal concentrations with internal dose of PFHxS

First, to obtain the internal dose of PFHxS, the UFA was applied to the rat model by finding the $C_{average,rat}$ resulting from repeated exposure to NOAEL.

As a result, $BE_{POD}$ was calculated as 4127.4 ng/mL. As a result of applying this to the UFH, as shown in FIG. 20, the internal dose in humans was derived as 412.7 ng/mL.

FIG. 20 is a graph comparing the internal dose of PFHxS and the blood concentration of PFHxS in the human body.

The internal concentrations of PFHxS in individual subjects were reported to be 2.29 ng/mL and 1.35 ng/mL for men and women, respectively, in Siheung, and 2.19 ng/mL and 1.75 ng/mL for men and women, respectively, in Daegu. Comparing this to the previously calculated internal dose of PFHxS (412.7 ng/mL), it may be seen that the actual blood levels of PFHxS in Koreans are low and consequently in the safe zone, as shown in FIG. 20.

## Claims

1. A physiologically based pharmacokinetic (PBPK) core model that is commonly applicable to two or more compounds, wherein a core structure of the two or more compounds comprises one or more selected from the group consisting of Formula 1 to Formula 3 below.

[Formula 1]

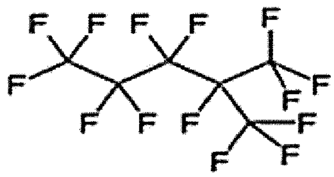

[Formula 2]

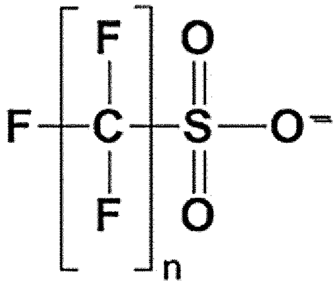

[Formula 3]

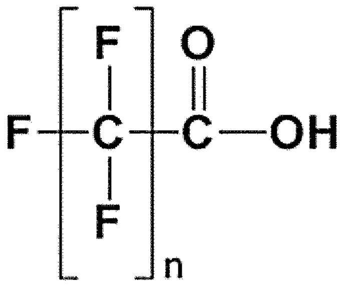

2. The PBPK core model of claim 1, wherein the two or more compounds are a member of the perfluorinated chemicals (PFCs) family.

3. The PBPK core model of claim 2, wherein the member of the perfluorinated chemicals (PFCs) family is one or more selected from the group consisting of compounds in the family of perfluorocarboxylic acids (PFCAs) and compounds in the family of perfluoroalkane sulfonates (PFSAs).

4. The PBPK core model of claim 1, wherein compartments of the PBPK core model comprises one or more selected from the group consisting of rich perfused tissue, slow perfused tissue, kidney, storage compartment, blood pool, gastrointestinal tract (G.I tract), and liver.

5. The PBPK core model of claim 1, wherein the PBPK core model comprises one or more mass balance differential equations selected from the group consisting of Equation 1 to Equation 11.

[Equation 1]

$$V_{bl} \cdot \frac{dC_{bl}}{dt} = Free \cdot \left(Q_{ri}\frac{C_{ri}}{K_{ri}} + Q_{sl}\frac{C_{sl}}{K_{sl}} + Q_{li}\frac{C_{li}}{K_{li}} + \frac{T_m C_{ki}}{K_t + C_{ki}}\right)$$
$$- Free \cdot (Q_{ri} + Q_{sl} + Q_{ki} + Q_{gi} + Q_{li}) C_{bl}$$

[Equation 2]

$$\frac{dA_{oral}}{dt} = -K_{abs}A_{oral}$$

[Equation 3]

$$A_{oral}(t=0) = dose \cdot (1 - F_u)$$

[Equation 4]

$$V_{gi}\frac{dC_{gi}}{dt} = Free \cdot (Q_{gi}C_{bl} - Q_{gi}\frac{C_{gi}}{K_{gi}} + Q_{li}\frac{C_{li}}{K_{li}} - K_f\frac{C_{gi}}{K_{gi}}) + K_{abs} \cdot A_{oral}$$

[Equation 5]

$$V_{li}\frac{dC_{li}}{dt} = Free \cdot (Q_{li}C_{bl} + Q_{gi}\frac{C_{gi}}{K_{gi}} - Q_{li}\frac{C_{li}}{K_{li}} - Q_{li}\frac{C_{li}}{K_{li}})$$

[Equation 6]

$$V_{ki}\frac{dC_{ki}}{dt} = Free \cdot Q_{ki}C_{bl} + \frac{T_mC_{fil}}{K_t + C_{fil}} - Q_{ki}C_{ki}$$

[Equation 7]

$$V_{fil}\frac{dC_{fil}}{dt} = Free \cdot Q_{fil}C_{bl} - \frac{T_mC_{fil}}{K_t + C_{fil}} - K_{st}C_{fil}$$

[Equation 8]

$$\frac{dA_{st}}{dt} = K_{st}C_{fil} - K_uA_{st}$$

[Equation 9]

$$\frac{dA_{urine}}{dt} = K_uA_{st}$$

[Equation 10]

$$\frac{dA_u}{dt} = K_u A_{st}$$

[Equation 11]

$$\frac{dA_f}{dt} = K_f \cdot Q_{gi} \frac{C_{gi}}{K_{gi}}$$

**6.** A method of providing information on external dose of a compound for a subject by using the physiologically based pharmacokinetic core model (PBPK core model) of claim 1.

**7.** A method of building a physiologically based pharmacokinetic core model (PBPK core model), comprising:

constructing an individual PBPK model of two or more compounds that have a common core structure;
selecting a compartment of a core model from a structure of the individual model; and
constructing a PBPK core model by using the compartment.

**8.** The method of building a PBPK core model of claim 7, further comprising:

calculating a physicochemical parameter of a compound of which dose is to be measured, a physiological parameter of the compound, or a biochemical parameter of the compound; and
establishing a mass balance differential equation for the selected compartment.

**9.** The method of constructing a PBPK core model of claim 7, further comprising: curve fitting a predicted time-blood concentration of a hazardous substance calculated from the constructed PBPK core model and a found time-blood concentration of the hazardous substance obtained from an animal experiment; and
verifying and validating the constructed PBPK core model.

**10.** The method of claim 7, wherein the selecting of a compartment of the core model is based on the structure of individual model and a pharmacokinetic profile, absorption, distribution, metabolism, excretion (ADME) characteristics, or a combination thereof.

**11.** The method of claim 7, wherein the compounds that have a common core structure are perfluorinated compounds (PFCs).

**12.** The method of claim 7, wherein the perfluorinated compounds are one or more selected from the group consisting of compounds of the perfluorocarboxylic acids (PFCAs) family, compounds of the perfluoroalkane sulfonates (PFSAs) family, and compounds of the per- and polyfluorinated substances (PFAS) family.

**13.** The method of claim 7, wherein the compartment of the PBPK core model comprises one or more selected from the group consisting of rich perfused tissue, slow perfused tissue, kidney, storage compartment, blood pool, gastrointestinal tract (G.I tract), and liver.

**14.** The method of claim 8, wherein the mass balance differential equation comprises one or more selected from the group consisting of the following Equation 1 to Equation 11.

[Equation 1]

$$V_{bl} \cdot \frac{dC_{bl}}{dt} = Free \cdot (Q_{ri}\frac{C_{ri}}{K_{ri}} + Q_{sl}\frac{C_{sl}}{K_{sl}} + Q_{li}\frac{C_{li}}{K_{li}} + \frac{T_m C_{ki}}{K_t + C_{ki}}) - Free \cdot (Q_{ri} + Q_{sl} + Q_{ki} + Q_{gi} + Q_{li})C_{bl}$$

[Equation 2]

$$\frac{dA_{oral}}{dt} = -K_{abs}A_{oral}$$

[Equation 3]

$$A_{oral}(t=0) = dose \cdot (1 - F_u)$$

[Equation 4]

$$V_{gi}\frac{dC_{gi}}{dt} = Free \cdot (Q_{gi}C_{bl} - Q_{gi}\frac{C_{gi}}{K_{gi}} + Q_{li}\frac{C_{li}}{K_{li}} - K_f\frac{C_{gi}}{K_{gi}}) + K_{abs} \cdot A_{oral}$$

[Equation 5]

$$V_{li}\frac{dC_{li}}{dt} = Free \cdot (Q_{li}C_{bl} + Q_{gi}\frac{C_{gi}}{K_{gi}} - Q_{li}\frac{C_{li}}{K_{li}} - Q_{li}\frac{C_{li}}{K_{li}})$$

[Equation 6]

$$V_{ki}\frac{dC_{ki}}{dt} = Free \cdot Q_{ki}C_{bl} + \frac{T_m C_{fil}}{K_t + C_{fil}} - Q_{ki}C_{ki}$$

[Equation 7]

$$V_{fil}\frac{dC_{fil}}{dt} = Free \cdot Q_{fil}C_{bl} - \frac{T_m C_{fil}}{K_t + C_{fil}} - K_{st}C_{fil}$$

[Equation 8]

$$\frac{dA_{st}}{dt} = K_{st}C_{fil} - K_u A_{st}$$

[Equation 9]

$$\frac{dA_{urine}}{dt} = K_u A_{st}$$

[Equation 10]

$$\frac{dA_u}{dt} = K_u A_{st}$$

[Equation 11]

$$\frac{dA_f}{dt} = K_f \cdot Q_{gi} \frac{C_{gi}}{K_{gi}}$$

FIG. 1

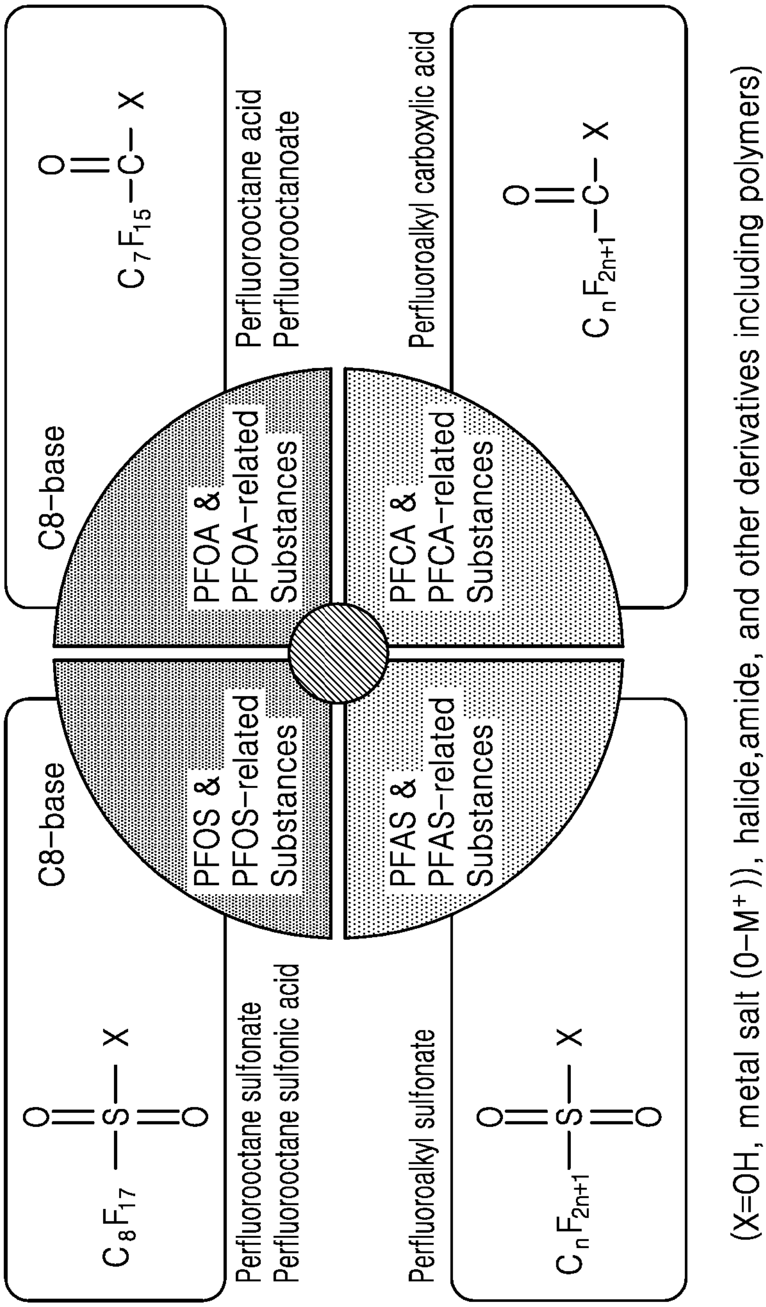
C8-base
PFOS &
PFOS-related
Substances
C8-base
PFOA &
PFOA-related
Substances
PFAS &
PFAS-related
Substances
PFCA &
PFCA-related
Substances
Perfluorooctane sulfonate
Perfluorooctane sulfonic acid
Perfluorooctane acid
Perfluorooctanoate
Perfluoroalkyl sulfonate
Perfluoroalkyl carboxylic acid
(X=OH, metal salt (0−M+)), halide,amide, and other derivatives including polymers)

FIG. 2

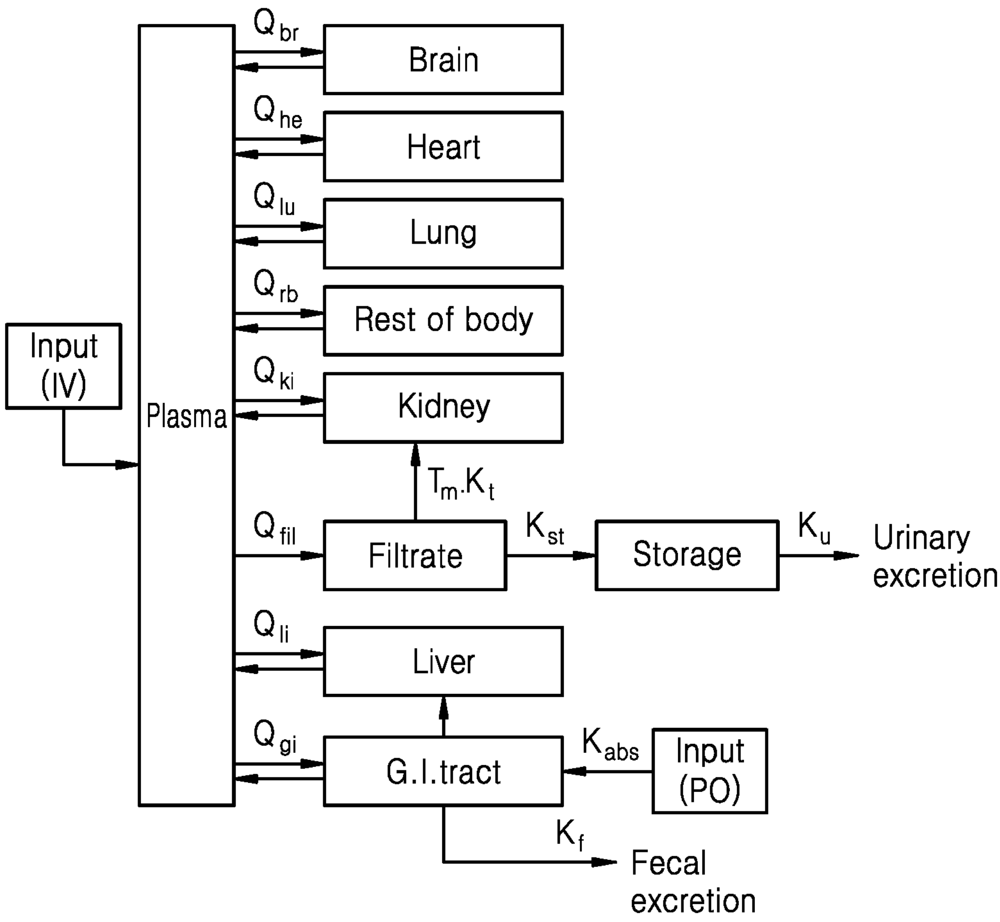
Input
(IV)
Plasma
Q br
Brain
Q he
Heart
Q lu
Lung
Q rb
Rest of body
Q ki
Kidney
Tm.Kt
Q fil
Filtrate
Kst
Storage
Ku
Urinary
excretion
Q li
Liver
Q gi
G.I.tract
Kabs
Input
(PO)
Kf
Fecal
excretion

# FIG. 3

```
;PFHxS PBPK model (male Rat)

STARTTIME = 0
STOPTIME = 350
DT = 0.1
DTOUT = 0.2

init Z1 = 0
init Z2 = 0
init Z3 = 0
init Z4 = 0
init Z5 = 0
        :init Z6 = 0
        :init Z7 = 0
init Z8 = 0
init Z9 = 0
init Z10 = 0
init Z11 = 0
        :init Z12 = 0
init Z13 = 0
init Z14 = 0
init Z15 = does*(1-Fu)
init Z16 = 0
```

INPUT INITIAL VALUE

CODING DIFFERENTIAL EQUATION

```
D/DT(Z1) = (Qbr*Z5*frBr+Qhe*Z4*frHe+Qlu*Z3*frLu+Qrb*Z2*frrb+Qki*Z8*frki+Qli*Z11*frli)/Vbl-(fr*(Qbr+Qhe+QLu+Qrb+Qki+Qli+Qgi+Qfil)*Z1)/Vbl    ;Plasma

D/DT(Z5) = (fr*Qbr*Z1-frBr*Qbr*Z5)/Vbr    :brain

D/DT(Z4) = (fr*Qhe*Z1-frHe*Qhe*Z4)/Vhe    :heart

D/DT(Z3) = (fr*QLu*Z1-frLu*Qlu*Z3)/Vlu    :lung

D/DT(Z2) = (fr*Qrb*Z1-frrb*Qrb*Z2)/Vrb    :rest of body

D/DT(Z8) = (fr*Qki*Z1+(Tm*Z16/(Kt+Z16))-frki*Qki*Z8)/Vki    :kidney

D/DT(Z16) = (fr*Qfil*Z1-(Tm*Z16/(Kt+Z16))-Kst*Z16)/Vfil    :filtrate

D/DT(Z9) = Kst*Z16·Ku*Z9    :storage

D/DT(Z11) = (fr*Qli*Z1+frGi*Qgi*Z10-frLi*0.94*Z11-frLi*Qli*Z11)/Vfil    :liver

D/DT(Z10) = (Kabs*Z15+fr*Qgi*Z1+frLi*0.94*Z11-frGi*Qgi*Z10-Kf*Z10*frGi)Vgi    :gestrointestinal
D/DT(Z13) = Ku*Z9    :unine

D/DT(Z14) = frGi*Kf*Z10    :feces

D/DT(Z15) = Kabs*Z15    :input
```

# FIG. 4

INPUT PHYSIOLOGICAL PARAMETER

Vbl = 20.4 :Volume of total blood (mL)
Vrb = 133.3 :Volume of rest of body (mL)
Vlu = 1 :Volume of lung (mL)
Vhe = 0.8 :Volume of heart (mL)
Vbr = 0.7 :Volume of brain (mL)
;Vad = 10 :Volume of adipose tissue (mL)
;Vmu = 122 :Volume of muscle (mL)
Vki = 1.8 :Volume of kidney (mL)
Vgi = 10 :Volume of G.I. (mL)
Vli = 8 :Volume of liver (mL)
;Vsp = 1.3 :Volume of spleen (mL)
Vfil = 0.045

Qrb = 510 :Blood flow to rest of body (mL/h)
Qlu = 2580 :Blood flow to lung (mL/h)
Qhe = 234 :Blood flow to heart (mL/h)
Qbr = 18 :Blood flow to brain (mL/h)
;Qad = 24 :Blood flow to adipose tissue (mL/h)
;Qmu = 450 :Blood flow to muscle (mL/h)
Qki = 520 :Blood flow to kidney (mL/h)
Qgi = 450 :Blood flow to G.I. (mL/h)
Qli = 828 :Blood flow to liver (mL/h)
;Qsp = 36 :Blood flow to spleen (mL/h)
Qfil = 3.09 :Qfil is not a blood flow, it is a clearance (Ml/h) from the plasma to the filtrate compartment

INPUT OTHER COMMANDS

Bloodpool = Z1
Rest of body = Z2
Lung = Z3
Heart = Z4
Brain = Z5
;Adipose = Z6
;Muscle = Z7
Kidney = Z8
Storage = Z9
GI = Z10
Liver = Z11
;Spleen = Z12
Urine = Z13
Feces = Z14
;display bloodpool, liver, Kidney, spleen, heart, lung, gi
Filtrate = Z16

RENAME TIME = T

PARTITION COEFFICIENT

Kli = 0.127 :Partition coefficient of liver
Krb = 0.027 :Partition coefficient of rest of body
Klu = 0.068 :Partition coefficient of lung
Khe = 0.033 :Partition coefficient of heart
Kbr = 0.005 :Partition coefficient of brain
;Kad = 0.037 :Partition coefficient of adipose tissue
;Kmu = 0.015 :Partition coefficient of muscle
Kki = 0.065 :Partition coefficient of kidney
Kgi = 0.029 :Partition coefficient of G.I.
;Ksp = 0.029 :Partition coefficient of spleen

PREDICTIVE PARAMETER

Tm = 1020 :Transport maximum (ng/h)
Kt = 11040 :Transport affinity constant
Ku = 0.92 :Rate constant to urine
Kf = 5.6 :Transport rate constant from intake to fecal elimination
Kabs = 0.23 :Oral absorption rate
Fu = 0.0001 :Fraction of dose that goes directly to feces
fr = 0.07 :Free fraction of PFHxS in blood
Kst = 152.9 :Kidney to delay compartment value

FIG. 5

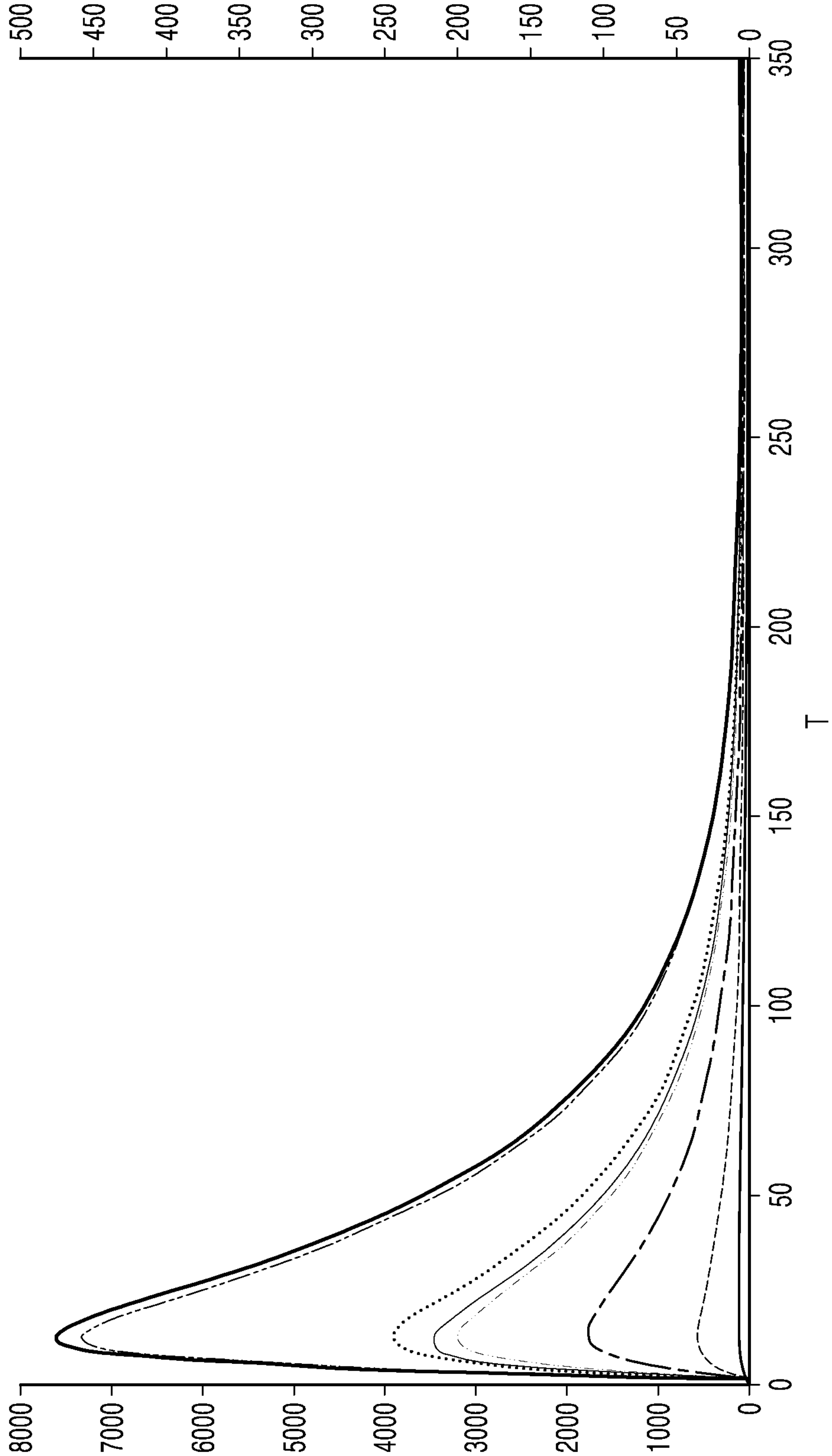
8000
7000
6000
5000
4000
3000
2000
1000
0
0
50
100
150
200
250
300
350
T
500
450
400
350
300
250
200
150
100
50
0

FIG. 6

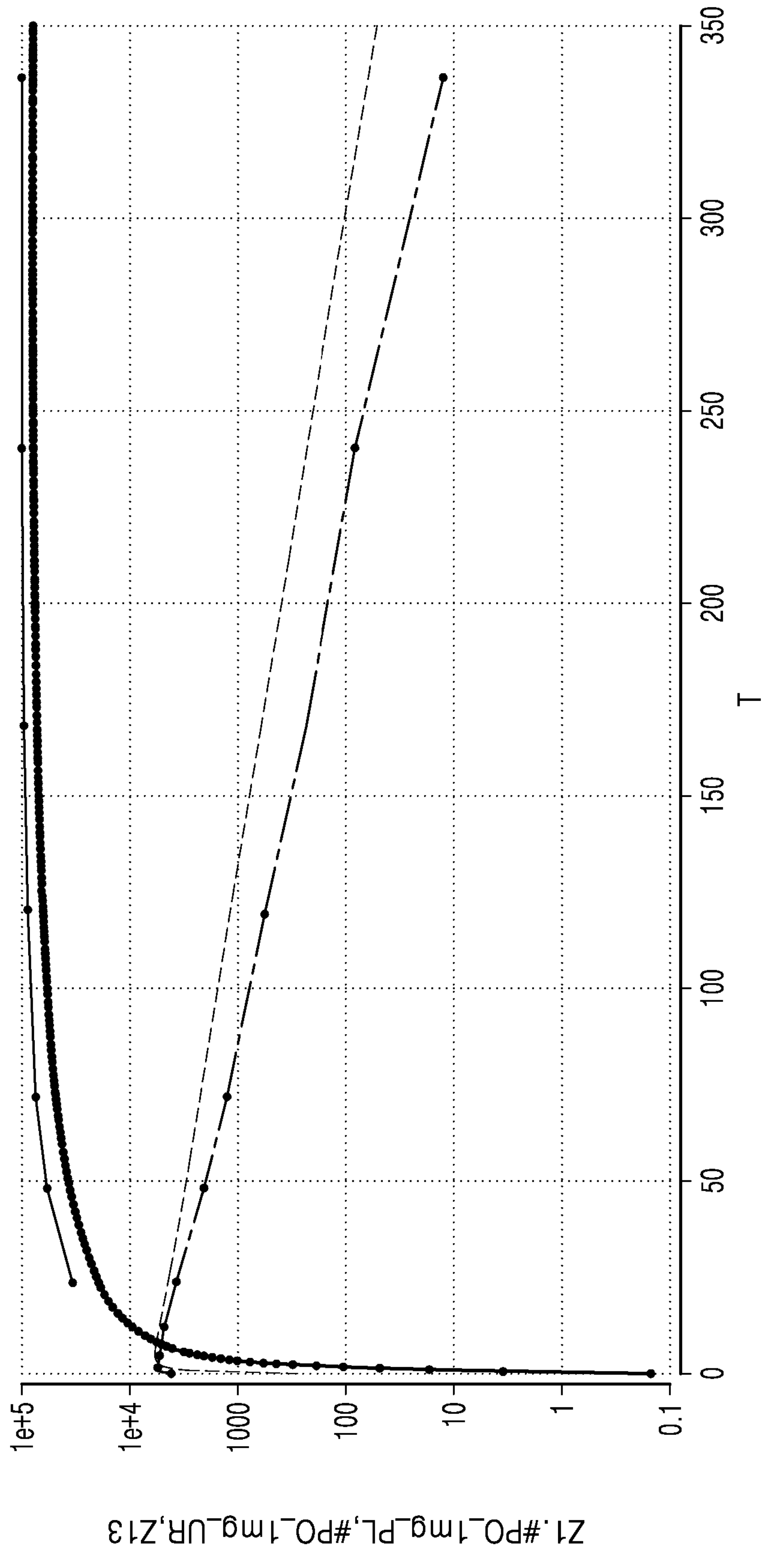

Z1.#PO_1mg_PL,#PO_1mg_UR,Z13
1e+5
1e+4
1000
100
10
1
0.1
0
50
100
150
200
250
300
350
T

# FIG. 7

INPUT PHYSIOLOGICAL PARAMETER

| | |
|---|---|
| Vbl = 20.4 | :Volume of total blood (mL) |
| Var = 6.8 | :Volume of artety (mL) |
| Vlu = 1 | :Volume of lung (mL) |
| Vhe = 0.8 | :Volume of heart (mL) |
| Vth = 0.7 | :Volume of thymus (mL) |
| Vad = 10 | :Volume of adipose tissue (mL) |
| Vmu = 122 | :Volume of muscle (mL) |
| Vki = 1.8 | :Volume of kidney (mL) |
| Vgi = 10 | :Volume of G.I. (mL) |
| Vli = 8 | :Volume of liver (mL) |
| Vsp = 1.3 | :Volume of spleen (mL) |
| Qve = 2580 | :Blood flow to vein (ML/h) |
| Qlu = 2580 | :Blood flow to lung (ML/h) |
| Qhe = 234 | :Blood flow to heart (ML/h) |
| Qth = 18 | :Blood flow to thymus (ML/h) |
| Qad = 24 | :Blood flow to adipose tissue (ML/h) |
| Qmu = 450 | :Blood flow to muscle (ML/h) |
| Qki = 520 | :Blood flow to kidney (ML/h) |
| Qgi = 450 | :Blood flow to G.I. (ML/h) |
| Qli = 828 | :Blood flow to liver (ML/h) |
| Qsp = 36 | :Blood flow to spleen (ML/h) |

INPUT OTHER COMMANDS

Bloodpool = Z1
Lung = Z3
Heart = Z4
Thymus = Z5
Adipose = Z6
Muscle = Z7
Kidney = Z8
Storage = Z9
GI = Z10
Liver = Z11
Spleen = Z12
Urine= Z13
Feces = Z14
display bloodpool, Adipose, Kidney, GI, Liver, Spleen, Lung, Heart, Thymus, Muscle

RENAME TIME = T

PARTITION COEFFICIENT

| | |
|---|---|
| Klu = 0.35 | :Partition coefficient of lung |
| Khe = 0.12 | :Partition coefficient of heart |
| Kth = 0.13 | :Partition coefficient of thymus |
| Kad = 0.09 | :Partition coefficient of adipose tissue |
| Kmu = 0.07 | :Partition coefficient of muscle |
| Kki = 1.04 | :Partition coefficient of kidney |
| Kgi = 0.10 | :Partition coefficient of G.I. |
| Kli = 0.84 | :Partition coefficient of liver |
| Ksp = 0.13 | :Partition coefficient of spleen |

PREDICTIVE PARAMETER

| | |
|---|---|
| Tm = 36210.3 | :Transport maximum (ng/h) |
| Kt = 913.389 | :Transport affinity constant |
| Ku = 0.02 | :Rate constant to urine |
| Kf = 4.622e+6 | :Transport rate constant from intake to fecal elimination |
| Ka = 31.3 | :Oral absorption rate |
| Fu = 0.01 | :Fraction of dose that goes directly to feces |
| fr = 0.064 | :Free fraction of PFOA in blood |
| Kst = 2.32832 | :Kidney to delay compartment value |

FIG. 8

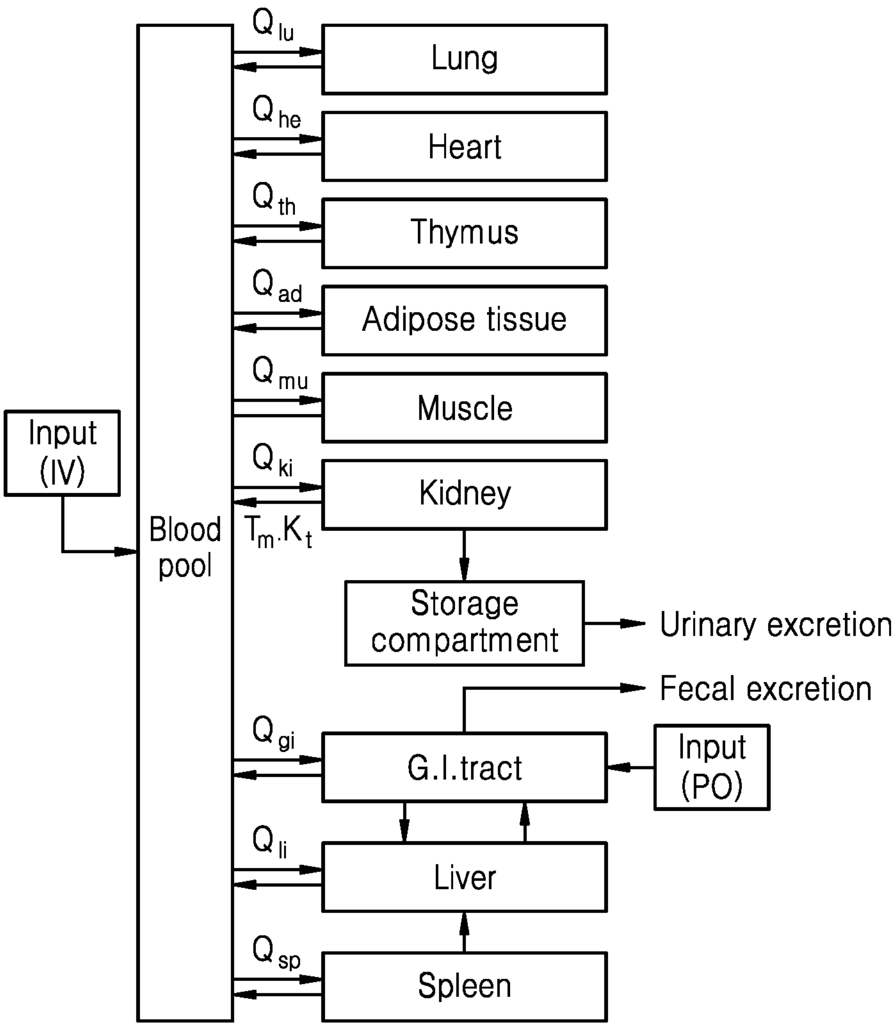
Input
(IV)
Blood
pool
Q lu
Lung
Q he
Heart
Q th
Thymus
Q ad
Adipose tissue
Q mu
Muscle
Q ki
Kidney
Tm.Kt
Storage
compartment
Urinary excretion
Fecal excretion
Q gi
G.I.tract
Input
(PO)
Q li
Liver
Q sp
Spleen

# FIG. 9

INPUT PHYSIOLOGICAL PARAMETER

| | |
|---|---|
| Vli = 0.00969 | :Volume of liver (L) |
| Vki = 0.01 | :Volume of kidney (L) |
| Vhe = 0.0008 | :Volume of heart (L) |
| Vlu = 0.001 | :Volume of lung (L) |
| Vad = 0.01 | :Volume of adipose tissue (L) |
| Vmu = 0.122 | :Volume of muscle (L) |
| Vsp = 0.0013 | :Volume of spleen (L) |
| Vgi = 0.01 | :Volume of G.I. (L) |
| Qli = 1.01196 | :Blood flow to vein (L/h) |
| Qki = 0.1548 | :Blood flow to kidney (L/h) |
| Qhe = 0.234 | :Blood flow to heart (L/h) |
| Qlu = 2.580 | :Blood flow to lung (L/h) |
| Qad = 0.024 | :Blood flow to adipose tissue (L/h) |
| Qmu = 0.45 | :Blood flow to muscle (L/h) |
| Qsp = 0.036 | :Blood flow to spleen (L/h) |
| Qgi = 0.45 | :Blood flow to G.I. (L/h) |

PARTITION COEFFICIENT

| | |
|---|---|
| Pli = 2.63 | :Partition coefficient of liver |
| Pki = 0.25 | :Partition coefficient of kidney |
| Phe = 0.04 | :Partition coefficient of heart |
| Plu = 0.17 | :Partition coefficient of lung |
| Pad = 0.01 | :Partition coefficient of adipose tissue |
| Pmu = 0.01 | :Partition coefficient of muscle |
| Psp = 0.07 | :Partition coefficient of spleen |
| Pgi = 0.05 | :Partition coefficient of G.I. |

PREDICTIVE PARAMETER

| | |
|---|---|
| Tm = 0.016 | :transport maximum (mg/h) |
| Kt = 13954 | :transporter disassociation constant (mg/L) |
| Ku = 0.00034 | :rate constant of the chemical to appear in urine (1/h) |
| Kabs = 82 | :oral absorption rate (1/h) |
| Kf = 0.0015 | :rate constant of unabsorbed chemical to appear in feces (1/h) |

FIG. 10

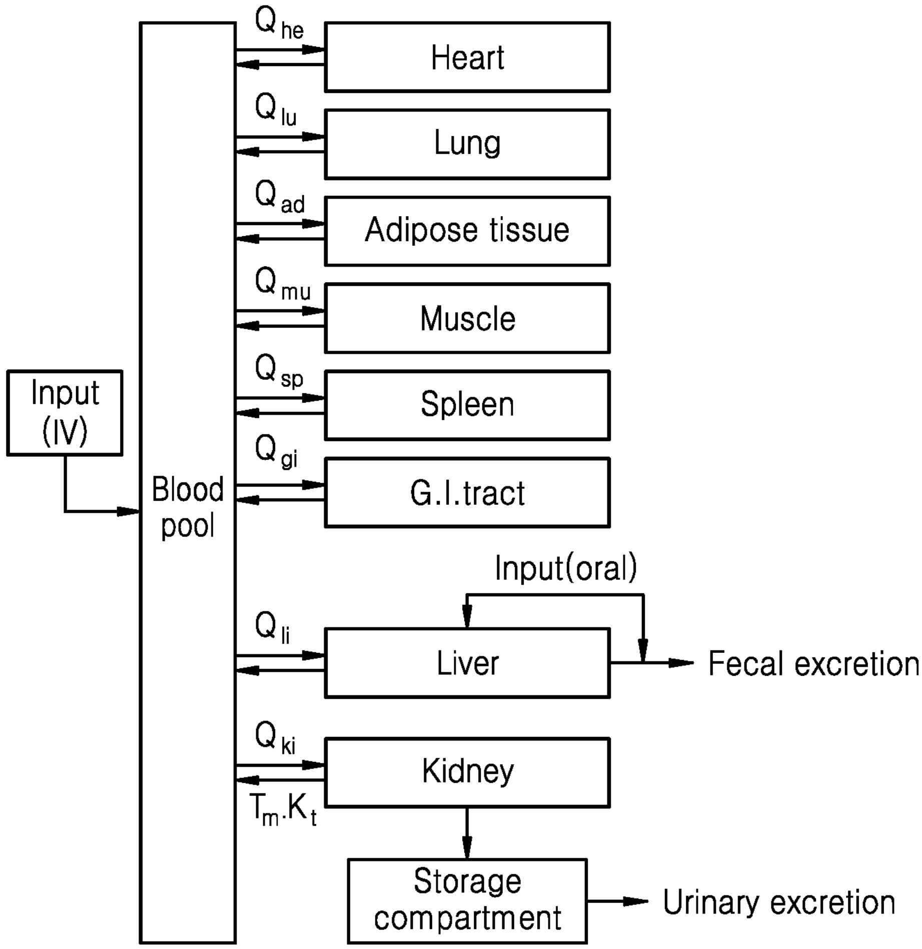
Input
(IV)
Blood
pool
Q he
Heart
Q lu
Lung
Q ad
Adipose tissue
Q mu
Muscle
Q sp
Spleen
Q gi
G.I.tract
Input(oral)
Q li
Liver
Fecal excretion
Q ki
Kidney
Tm.Kt
Storage
compartment
Urinary excretion

FIG. 11

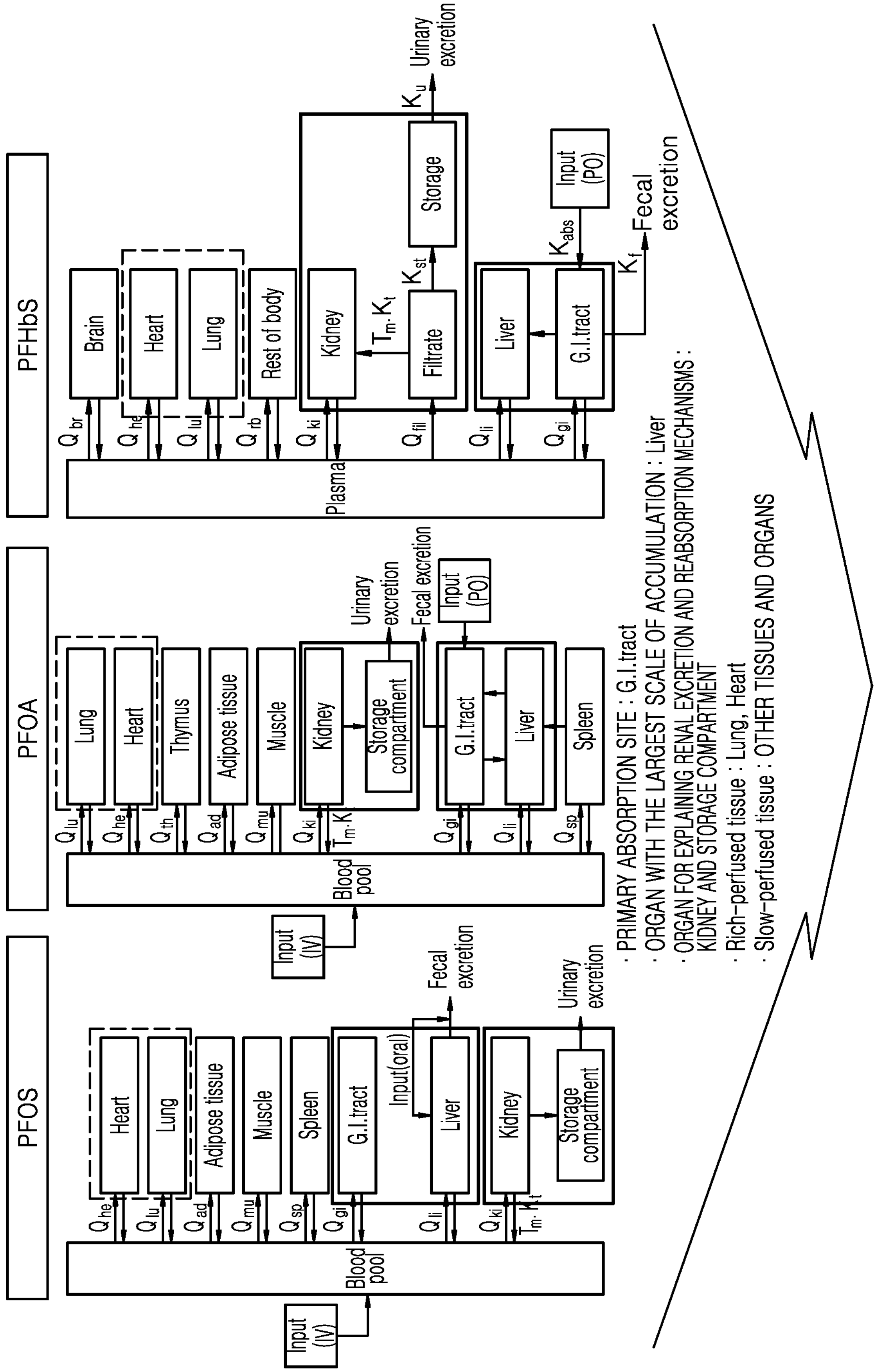
PFOS
PFOA
PFHbS
Input (IV)
Blood pool
Plasma
Heart
Lung
Adipose tissue
Muscle
Spleen
G.I.tract
Liver
Kidney
Storage compartment
Input(oral)
Fecal excretion
Urinary excretion
Thymus
Input (PO)
Brain
Rest of body
Filtrate
Storage
Fecal excretion
· PRIMARY ABSORPTION SITE : G.I.tract
· ORGAN WITH THE LARGEST SCALE OF ACCUMULATION : Liver
· ORGAN FOR EXPLAINING RENAL EXCRETION AND REABSORPTION MECHANISMS : KIDNEY AND STORAGE COMPARTMENT
· Rich-perfused tissue : Lung, Heart
· Slow-perfused tissue : OTHER TISSUES AND ORGANS

FIG. 12

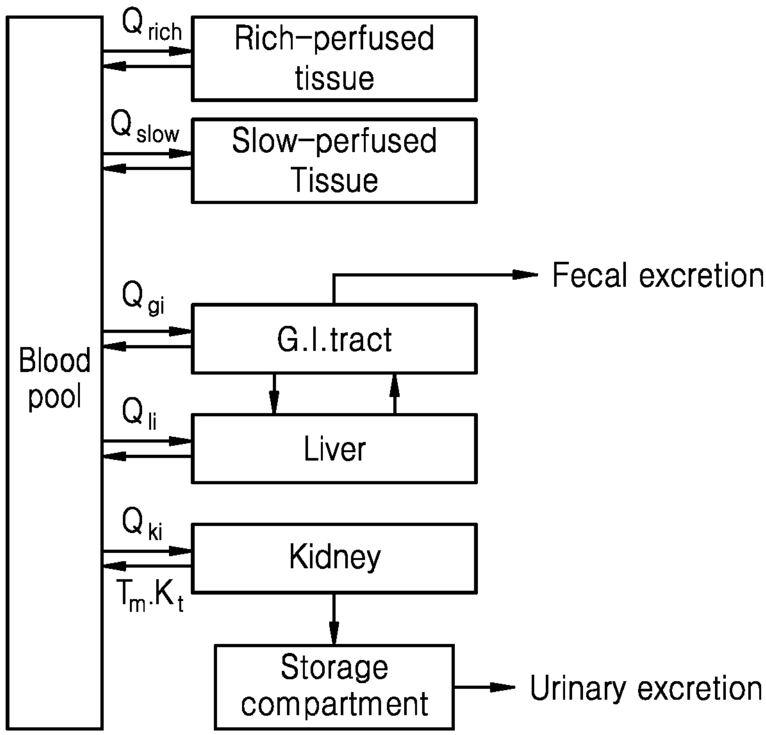
Blood pool
Q rich
Rich-perfused tissue
Q slow
Slow-perfused Tissue
Q gi
G.I.tract
Fecal excretion
Q li
Liver
Q ki
Kidney
Tm.Kt
Storage compartment
Urinary excretion

# FIG. 13

## LC-MS/MS conditions

· HPLC - Acquity UPLC system

| | |
|---|---|
| Column | Halo $C_{18}$ column (2.1 x 50 mm, 2.7 μm, Advancedmaterialstechnology) |
| Mobile Phase (Gradient) | (A) 2mM ammonium acetate in water<br>(B) Acetonitrile |
| Column temperature | 40±5°C |
| Flow rate | 0.2 mL/min |
| Injection volume | 2 μL |

| | Time (min) | Flow rate(mL/min) | A (%) | B (%) |
|---|---|---|---|---|
| 1 | 0.0 | 0.2 | 70 | 30 |
| 2 | 0.5 | 0.2 | 70 | 30 |
| 3 | 2.0 | 0.2 | 20 | 80 |
| 4 | 2.1 | 0.2 | 10 | 90 |
| 5 | 3.4 | 0.2 | 10 | 90 |
| 6 | 3.5 | 0.2 | 70 | 30 |
| 7 | 4.5 | 0.2 | 70 | 30 |

· MS/MS - Waters Xevo® TQ-S
Polarity: Electrospray ionization negative mode

| Analyte | MRM ion | Cone Voltage (V) | Collision Energy (eV) |
|---|---|---|---|
| PFHxS | 398.9/79.6 | 20 | 35 |
| MPFHxS (IS) | 402.9/102.9 | 20 | 35 |
| PFNA | 463.0/419.0 | 10 | 10 |
| MPFNA (IS) | 468.0/423.1 | 20 | 10 |
| PFDA | 513.0/469.0 | 10 | 10 |
| MPFDA (IS) | 515.1/470.0 | 20 | 10 |

## Sample preparation

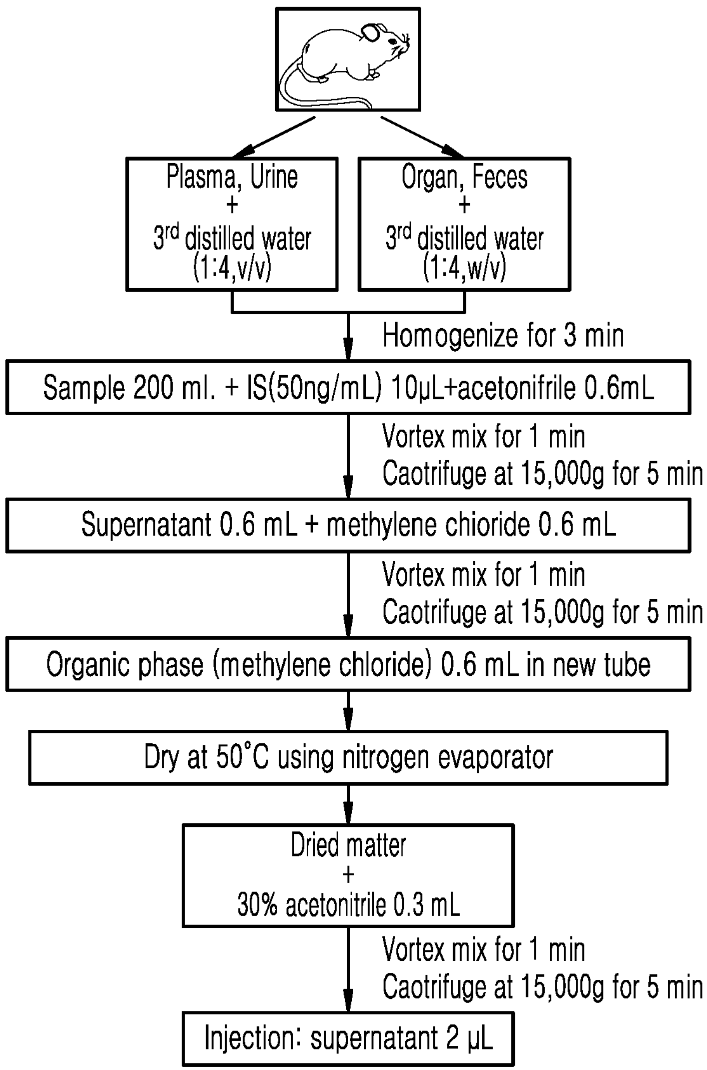

# FIG. 14

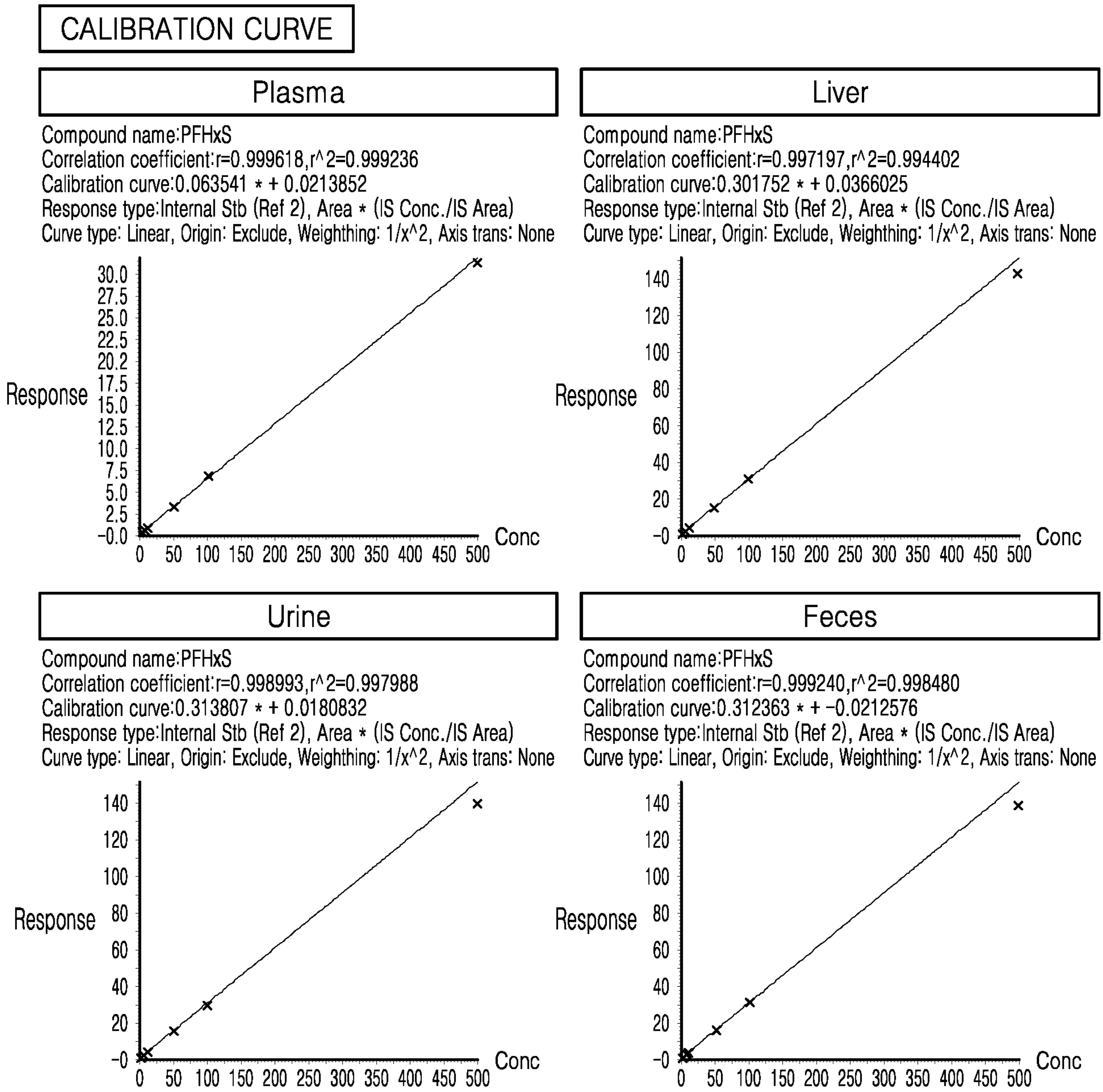


ACCURACY AND PRECISION

| | Intra (n=5) | | Inter (n=5) | |
|---|---|---|---|---|
| | Precision (%) | Accuracy (%) | Precision (%) | Accuracy (%) |
| Plasma | 3.66 - 6.85 | 92.09 - 104.67 | 4.07 - 6.43 | 97.53 - 105.65 |
| Liver | 1.31 - 6.79 | 93.66 - 105.33 | 3.77 - 12.22 | 91.83 - 103.10 |
| Urine | 0.59 - 1.45 | 96.46 - 102.00 | 1.41 - 11.87 | 95.93 - 102.03 |
| Feces | 1.65 - 7.71 | 94.04 - 106.62 | 1.52 - 4.48 | 95.90 - 101.89 |

# FIG. 15

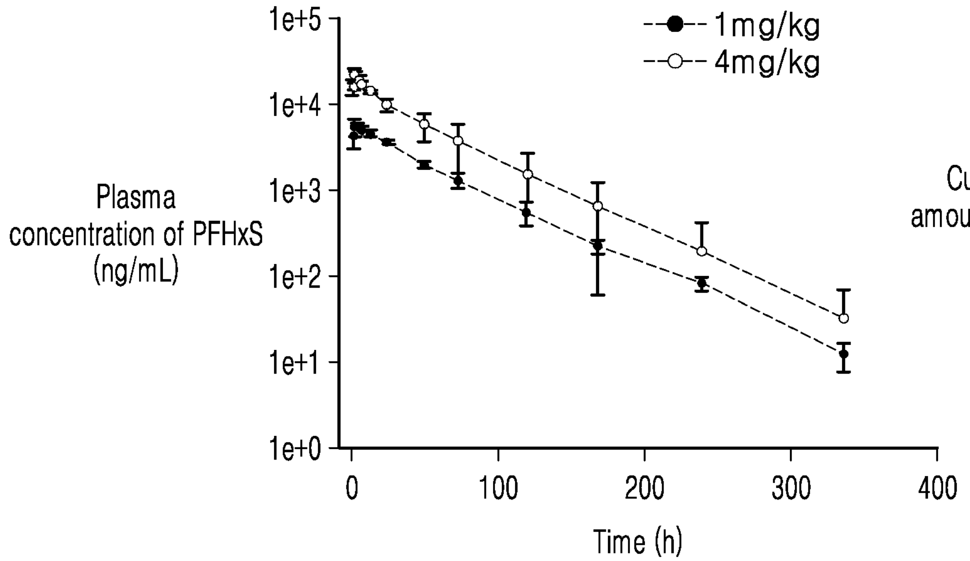


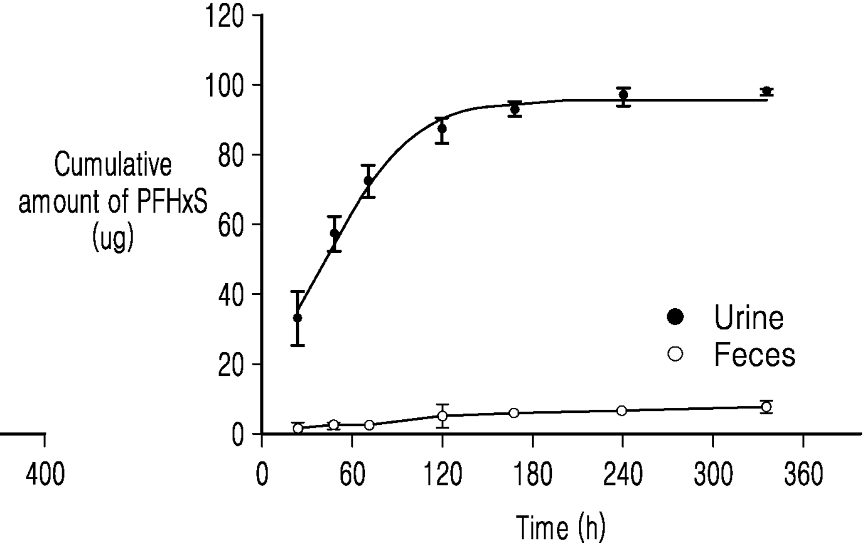


BLOOD CONCENTRATION-TIME CURVE OF PFHxS FOLLOWING ORAL ADMINISTRATION

CUMULATIVE AMOUNT OF PFHxS IN URINE AND FECES FOLLOWING ORAL ADMINISTRATION

BLOOD CONCENTRATION-TIME CURVE OF PFHxS FOLLOWING ORAL ADMINISTRATION

| Parameters | 1 mg/kg | 4 mg/kg |
|---|---|---|
| $t_{1/2}$(hr) | 38.45 ± 2.01 | 40.45 ± 1.40 |
| $ta_{1/2}$(hr) | 0.41 ± 0.09 | 0.45 ± 2.21 |
| $C_{max}$(μg/mL) | 5.50 ± 0.09 | 17.45 ± 0.48 |
| $AUC_{inf}$ (μg·hr/mL) | 296.12 ± 19.35 | 1471.74 ± 57.37 |
| Vd/F (L/kg) | 187.34 ± 3.49 | 158.60 ± 7.78 |
| CL/F (mL/hr/kg) | 3.34 ± 9.35 | 2.72 ± 0.34 |

# FIG. 16

| Parameters | Rat | Human | Unit |
|---|---|---|---|
| Kp_rest of body | 0.005 | 0.005 | |
| Kp_liver | 0.063 | 0.063 | |
| Kp_lung | 0.044 | 0.044 | |
| Kp_heart | 0.027 | 0.027 | |
| Kp_adipose tissue | 0.013 | 0.013 | $k_{human} = k_{rat} \cdot \left(\frac{Weight_{human}}{Weight_{rat}}\right)^{-0.25}$ |
| Kp_kidney | 0.062 | 0.062 | |
| Kp_G.I.tract | 0.018 | 0.018 | |
| Kp_spleen | 0.014 | 0.014 | |
| Tm (estimated)<br>(Transport maximum) | 300 | 300 | ng/h |
| Kt (estimated)<br>(Transport affinity constant) | 500 | 500 | μg/h |
| Ku (estimated)<br>(Rate constant to urine) | 0.20 | 0.049 | $hr^{-1}$ |
| Kf (estimated)<br>(Transfer rate constant from Intake to Inca) | 160 | 39.114 | $hr^{-1}$ |
| Kabs (estimated)<br>(Oral absorption rate) | 2.20 | 0.538 | $hr^{-1}$ |
| Fu (estimated)<br>(Fraction of dose that goes directly to feces) | 0.001 | 0.001 | |
| $F_{free}$ (estimated)<br>(Free fraction) | 0.00042 | 0.00042 | |
| Kst (estimated)<br>(kidney to delay compartment value) | 180.5 | 44.125 | $hr^{-1}$ |

FIG. 17

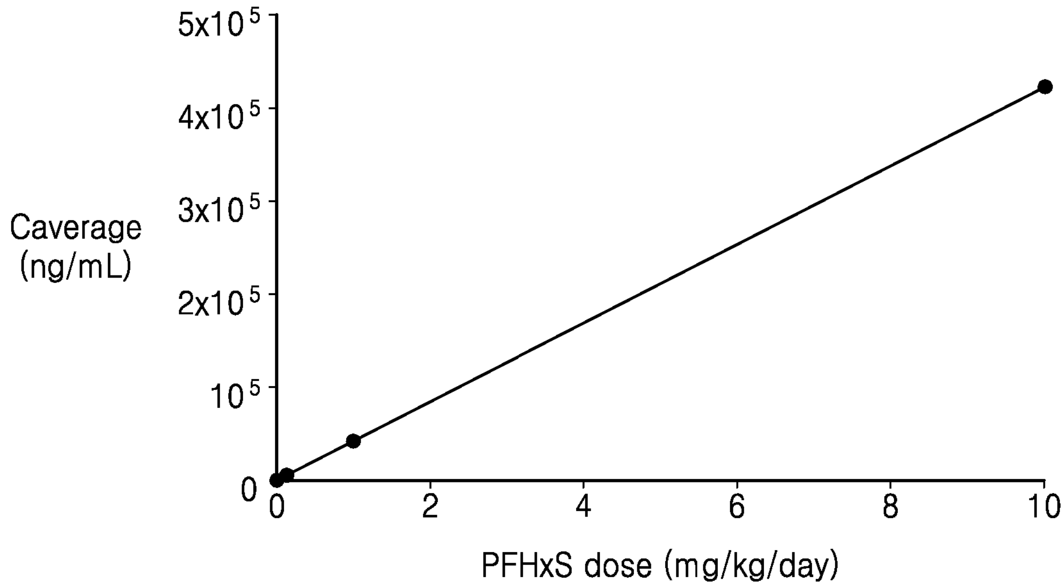
5x10^5
4x10^5
3x10^5
2x10^5
10^5
0
Caverage
(ng/mL)
0
2
4
6
8
10
PFHxS dose (mg/kg/day)

FIG. 18

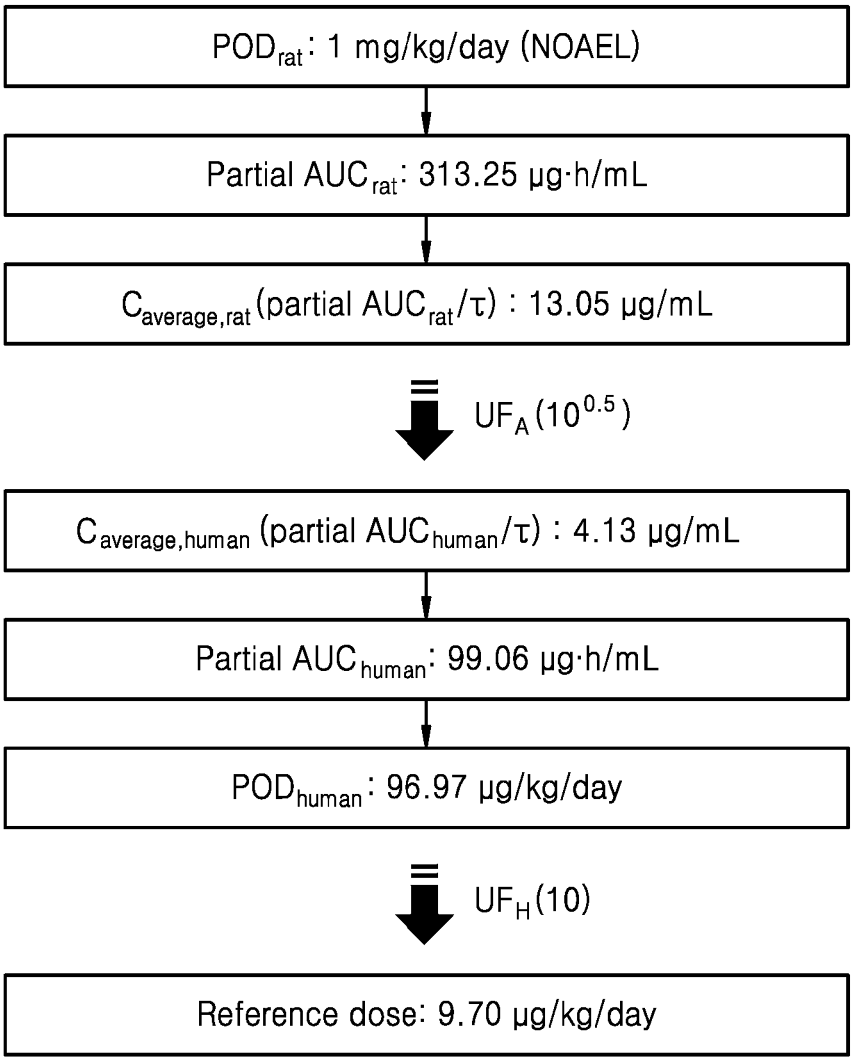
$POD_{rat}$: 1 mg/kg/day (NOAEL)
Partial $AUC_{rat}$: 313.25 μg·h/mL
$C_{average,rat}$ (partial $AUC_{rat}/\tau$) : 13.05 μg/mL
$UF_A$ ($10^{0.5}$)
$C_{average,human}$ (partial $AUC_{human}/\tau$) : 4.13 μg/mL
Partial $AUC_{human}$: 99.06 μg·h/mL
$POD_{human}$: 96.97 μg/kg/day
$UF_H$(10)
Reference dose: 9.70 μg/kg/day

FIG. 19

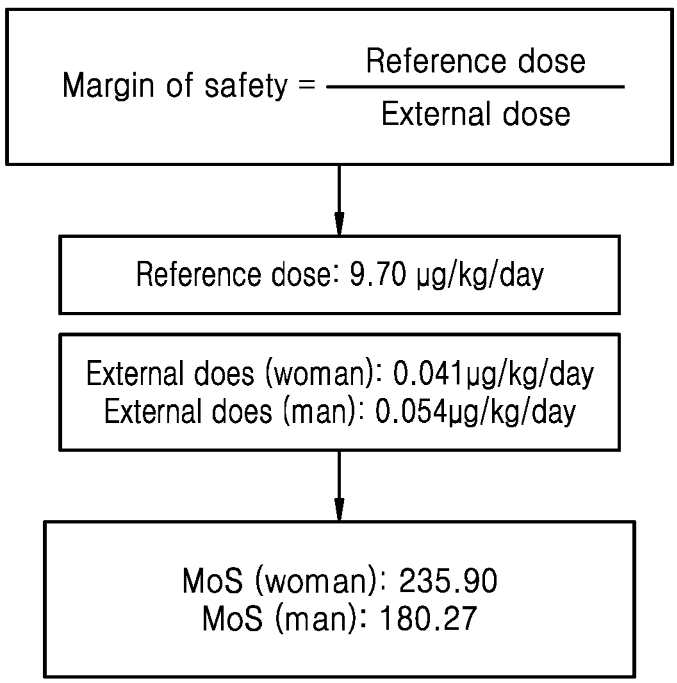
Margin of safety = Reference dose / External dose
Reference dose: 9.70 μg/kg/day
External does (woman): 0.041μg/kg/day
External does (man): 0.054μg/kg/day
MoS (woman): 235.90
MoS (man): 180.27

# FIG. 20

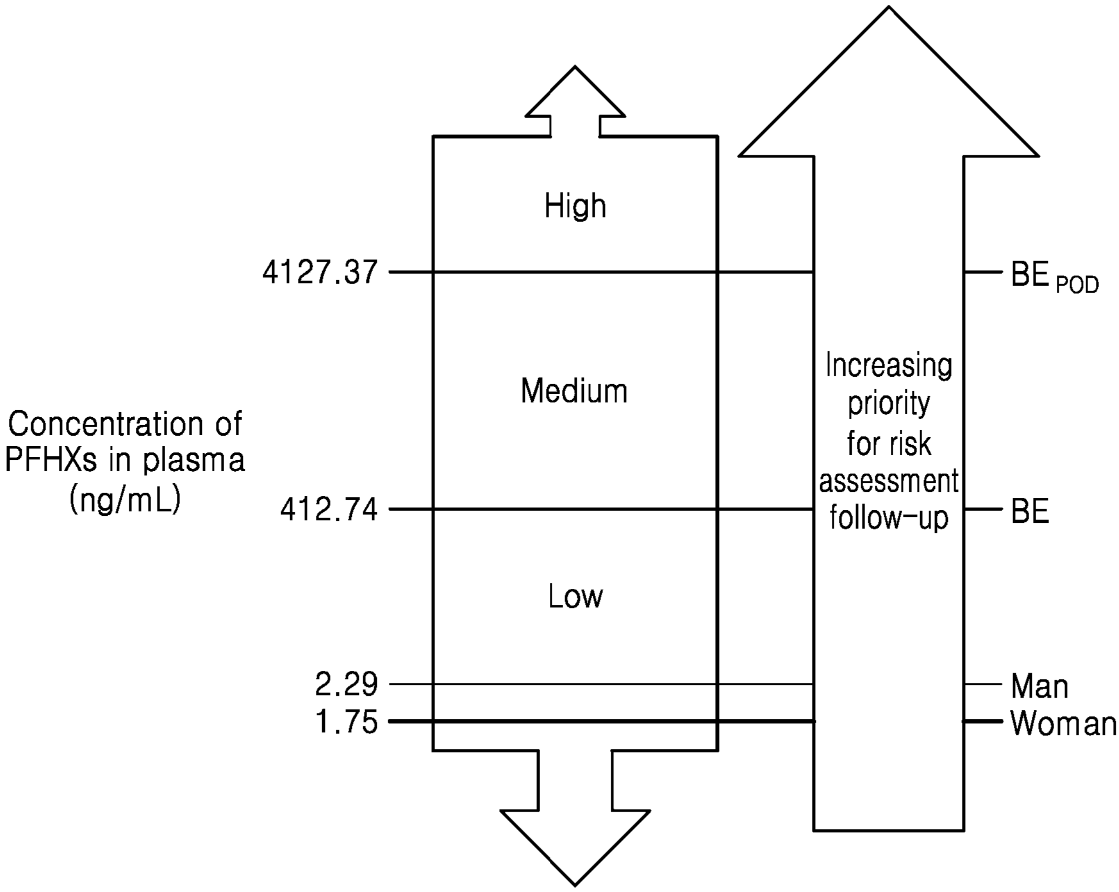

Concentration of PFHXs in plasma (ng/mL)
High
4127.37
Medium
412.74
Low
2.29
1.75
Increasing priority for risk assessment follow-up
BE POD
BE
Man
Woman

FIG. 21

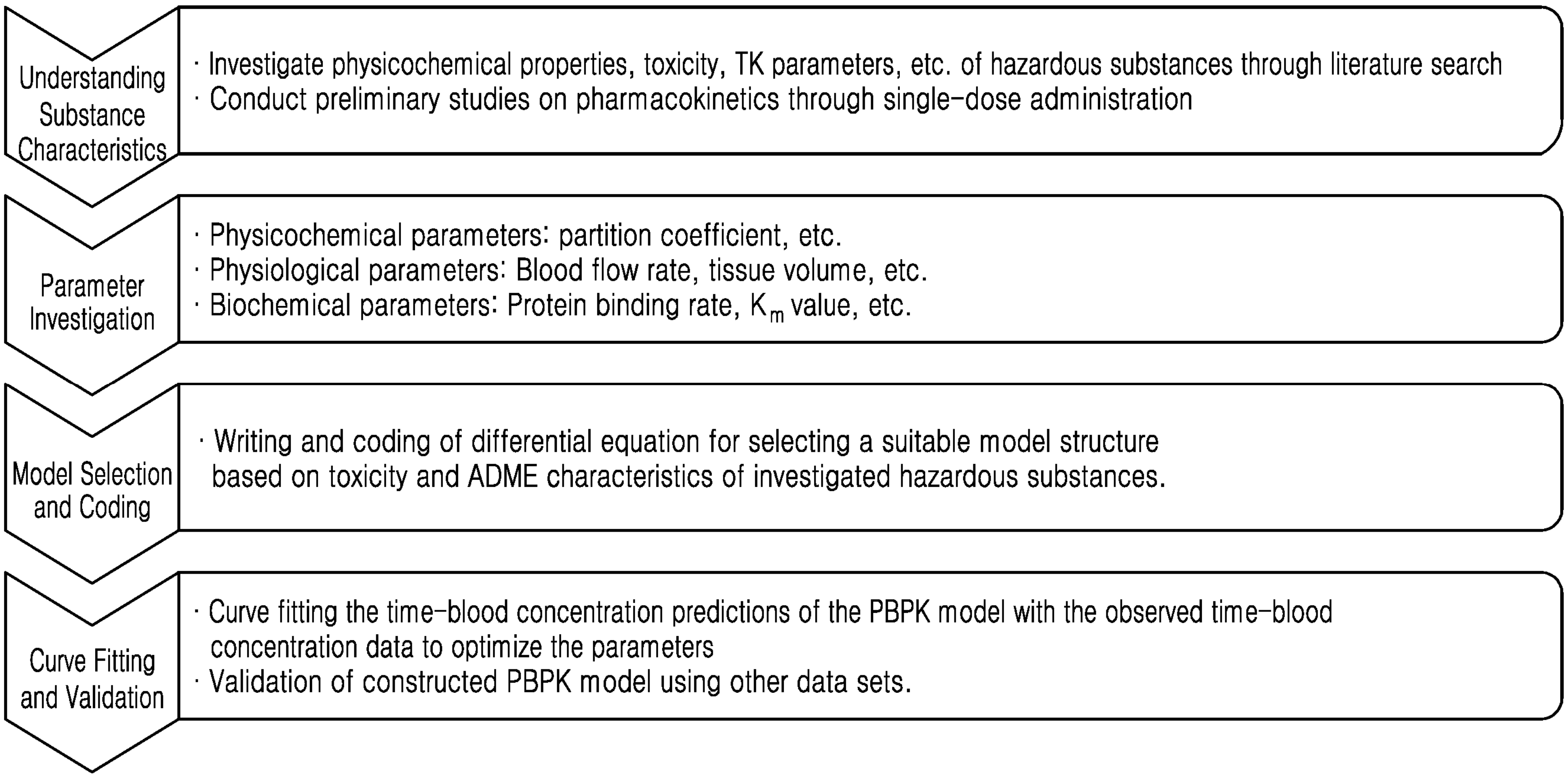
Understanding Substance Characteristics
· Investigate physicochemical properties, toxicity, TK parameters, etc. of hazardous substances through literature search
· Conduct preliminary studies on pharmacokinetics through single-dose administration
Parameter Investigation
· Physicochemical parameters: partition coefficient, etc.
· Physiological parameters: Blood flow rate, tissue volume, etc.
· Biochemical parameters: Protein binding rate, $K_m$ value, etc.
Model Selection and Coding
· Writing and coding of differential equation for selecting a suitable model structure based on toxicity and ADME characteristics of investigated hazardous substances.
Curve Fitting and Validation
· Curve fitting the time-blood concentration predictions of the PBPK model with the observed time-blood concentration data to optimize the parameters
· Validation of constructed PBPK model using other data sets.

## INTERNATIONAL SEARCH REPORT

International application No.
**PCT/KR2021/014995**

**A. CLASSIFICATION OF SUBJECT MATTER**

**G16C 20/50**(2019.01)i; **G16C 20/10**(2019.01)i; **G16C 20/30**(2019.01)i; **G16C 60/00**(2019.01)i

According to International Patent Classification (IPC) or to both national classification and IPC

**B. FIELDS SEARCHED**

Minimum documentation searched (classification system followed by classification symbols)

G16C 20/50(2019.01); G01N 33/00(2006.01); G16B 40/00(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생리학적 약물동태학 핵심 모델(physiologically based pharmacokinetic core model; PBPK core model), 과불화합물(perfluorinated chemicals; PFCs)

**C. DOCUMENTS CONSIDERED TO BE RELEVANT**

| Category* | Citation of document, with indication, where appropriate, of the relevant passages | Relevant to claim No. |
|---|---|---|
| X<br>Y | KIM, S.-J. et al. Exploring sex differences in human health risk assessment for PFNA and PFDA using a PBPK model. Archives of toxicology. 2019, vol. 93, pp. 311-330.<br>See abstract; pages 313-328; and figures 1-2 and 7-8. | 1-3,6-7,9-12<br>4-5,8,13-14 |
| Y | VERNER, M.-A. et al. Physiologically Based Pharmacokinetic Modeling of Persistent Organic Pollutants for Lifetime Exposure Assessment: A New Tool in Breast Cancer Epidemiologic Studies. Environmental Health Perspectives. 2008, vol. 116, no. 7, pp. 886-892.<br>See abstract; and pages 886-888. | 4,8,13-14 |
| Y | CHOWDHURY, M. M. et al. A Physiologically Based Pharmacokinetic Model for Absorption and Distribution of Imatinib in Human Body. Bulletin of the Korean Chemical Society. 2011, vol. 32, no. 11, pp. 3967-3972.<br>See abstract; and pages 3968-3971. | 5,8,14 |
| A | CN 105334292 A (YELLOW SEA FISHERIES RESEARCH INSTITUTE CHINESE ACADEMY OF FISHERY SCIENCES) 17 February 2016 (2016-02-17)<br>See entire document. | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 January 2022** | **28 January 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/014995**

**C. DOCUMENTS CONSIDERED TO BE RELEVANT**

| Category* | Citation of document, with indication, where appropriate, of the relevant passages | Relevant to claim No. |
|---|---|---|
| A | KR 10-2019-0109477 A (SHIRE HUMAN GENETIC THERAPIES, INC.) 25 September 2019 (2019-09-25) See entire document. | 1-14 |

Form PCT/ISA/210 (second sheet) (July 2019)

**INTERNATIONAL SEARCH REPORT**
**Information on patent family members**

International application No.
**PCT/KR2021/014995**

| Patent document cited in search report | Publication date (day/month/year) | Patent family member(s) | Publication date (day/month/year) |
|---|---|---|---|
| CN 105334292 A | 17 February 2016 | None | |
| KR 10-2019-0109477 A | 25 September 2019 | AR 110909 A1 | 15 May 2019 |
| | | AU 2018-213055 A1 | 15 August 2019 |
| | | BR 112019015213 A2 | 14 April 2020 |
| | | CA 3051244 A1 | 02 August 2018 |
| | | CL 2019002084 A1 | 08 May 2020 |
| | | CN 110383388 A | 25 October 2019 |
| | | CO 2019008885 A2 | 17 January 2020 |
| | | EA 202190788 A1 | 29 October 2021 |
| | | EP 3574423 A1 | 04 December 2019 |
| | | EP 3574423 A4 | 14 October 2020 |
| | | IL 268147 A | 26 September 2019 |
| | | JP 2020-510899 A | 09 April 2020 |
| | | JP 2021-108135 A | 29 July 2021 |
| | | JP 6860134 B2 | 14 April 2021 |
| | | KR 10-2021-0034689 A | 30 March 2021 |
| | | KR 10-2021-0088750 A | 14 July 2021 |
| | | KR 10-2021-0130258 A | 29 October 2021 |
| | | KR 10-2233784 B1 | 29 March 2021 |
| | | KR 10-2277321 B1 | 13 July 2021 |
| | | KR 10-2318205 B1 | 26 October 2021 |
| | | MA 46492 A1 | 29 November 2019 |
| | | MA 46492 B1 | 30 April 2020 |
| | | MX 2019008797 A | 11 September 2019 |
| | | PH 12019501752 A1 | 16 September 2019 |
| | | SG 11201906837 A | 27 August 2019 |
| | | US 10896749 B2 | 19 January 2021 |
| | | US 2018-0218782 A1 | 02 August 2018 |
| | | US 2021-0098103 A1 | 01 April 2021 |
| | | WO 2018-140373 A1 | 02 August 2018 |
| | | ZA 201904832 B | 23 December 2020 |

Form PCT/ISA/210 (patent family annex) (July 2019)